# EUROPEAN PATENT APPLICATION

(11) **EP 4 302 593 A1**
(43) Date of publication of application: **10.01.2024**
(21) Application number: 22763252.8
(22) Date of filing: 01.03.2022
(51) Int. Cl.: A01H 6/82, A01H 1/00, A01H 5/00, A23L 19/00, C12N 5/04, C12N 5/10, C12N 15/09, C12N 15/29, C12N 15/52

(54) **SOLANACEOUS PLANT RESISTANT TO BEGOMOVIRUS GENUS VIRUS CAUSING YELLOW LEAF CURL SYMPTOMS IN TOMATOES, SOLANACEOUS PLANT CELL, AND METHOD FOR PRODUCING SOLANACEOUS PLANT**

(30) Priority: 04.03.2021 JP 2021034360; 29.07.2021 JP 2021124112
(71) Applicant: Kikkoman Corporation, Noda-shi, Chiba 278-8601 (JP); National University Corporation Hokkaido University, Sapporo-shi, Hokkaido 060-0808 (JP)
(72) Inventor: ATARASHI, Hiroki, Noda-shi, Chiba 278-8601 (JP); KWON, Joon, Sapporo-shi, Hokkaido 060-0808 (JP); NAKAHARA, Kenji, Sapporo-shi, Hokkaido 060-0808 (JP)
(74) Representative: Eisenführ Speiser
(86) International application number: PCT/JP2022/008603
(87) International publication number: WO 2022/186197

(57) **Abstract**

The present invention addresses the problem of providing: a solanaceous plant resistant to a Begomovirus genus virus causing yellow leaf curl symptoms in tomatoes, the solanaceous plant having the property of inhibiting infection by the Begomovirus genus virus causing yellow leaf curl symptoms in tomatoes and the property of suppressing the proliferation of the virus after infection and/or the manifestation of the symptoms of infection with the virus; a solanaceous plant cell; and a method for producing a solanaceous plant. The problem is solved by providing a solanaceous plant having resistance to the aforementioned virus, wherein: at least one gene, which is selected from the group consisting of the DCL3 gene being a silencing-related dicer gene, the 4CL06 gene being a 4-coumaric acid CoA ligase gene, the RLK2 gene being a receptor-like ligase gene, and homologous genes thereof, has a mutation; and, as a result of the mutation, the expression of the gene having the mutation is suppressed or a protein encoded by the gene having the mutation is non-functional to the virus.

## Description

### Technical Field

The present disclosure relates to a solanaceous plant resistant to a virus of genus *Begomovirus* causing tomato yellow leaf curl symptoms, a solanaceous plant cell, and a method for producing the solanaceous plant.

### Background Art

Tomato yellow leaf curl virus (Tomato yellow leaf curl virus; hereinafter, may be abbreviated to "TYLCV"), a representative virus causing tomato yellow leaf curl disease, is one of a relatively new plant virus found in Israel in 1964. TYLCV and many other related species of viruses belonging to genus *Begomovirus* (the genus where TYLCV belongs) are found worldwide and are causing damages to various plants and crops mainly in tropical, subtropical and temperate regions. Distribution of TYLCV-related viruses is diverse and for example, Tomato yellow leaf curl Sardinia virus, a related viral species of TYLCV, is found only in Mediterranean regions. On the other hand, TYLCV is prevailing worldwide mainly in tomato-producing areas (see Non-Patent Literature (hereinafter, abbreviated NPL) 1).

In Japan, tomato yellow leaf curl disease caused by TYLCV were found simultaneously in Nagasaki, Aichi and Shizuoka prefectures in 1996 and, thereafter, occurrence of the disease is spreading rapidly in green house tomato producing areas. Especially from year 2000 onwards, occurrence of the disease is enormous in Kyushu area which is a major production area for raw eating tomatoes, and there is a continuous increase in number of farmers in which all of their cultivated tomatoes suffer from TYLCV damage. Each local government is issuing strict alerts to the farmers and conducting thorough TYLCV control by curbing insect vectors with pesticide, etc., but the occurrence of TYLCV damage is still continuing and spreading throughout the country. Even in 2016, TYLCV was a disease causing largest financial damage in tomato production.

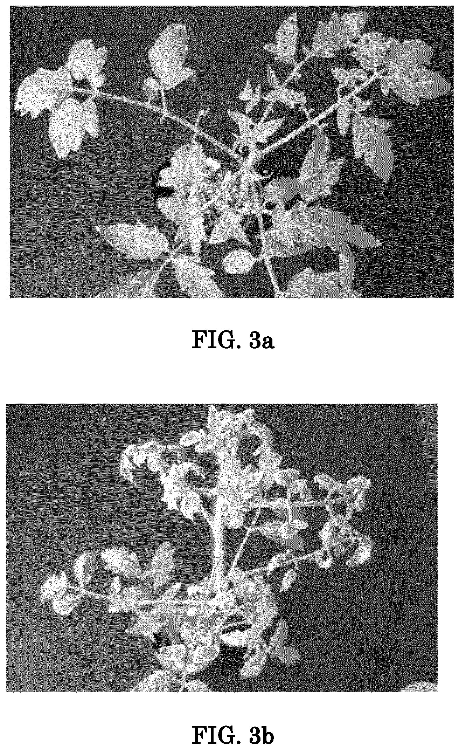

Symptoms of the tomato yellow leaf curl disease start with yellowing of tomato leaves and, then, edges of the leaves gradually curl (curve) downwards and end in malformation (see, for example, Figure 3(b)). When the symptoms become severe, a whole plant looks like permed head. Although fruits show no symptoms, effects of tomato plant infected with TYLCV in early stage of cultivation result in a harvest of only up to second or third fruit cluster, and make very large damage of 70 to 80% loss of fruit yield.

Tomato yellow leaf curl disease is transmitted by whitefly (Bemisia tabaci (Gennadius)), an insect vector for TYLCV, which permanently spreads.

Further, there is no effective anti-viral pesticide against the plant viruses *per se.* Conventional methods for controlling plant viruses are spraying of an insecticide against insect vectors transmitting the viruses, use of insect proof nets or insect evasion materials for physically avoiding invasion of insect vector, soil disinfestation, removal of infected plants, sterilization of cultivation tool, use of barrier plants, and breeding of virus resistant crops.

Control of TYLCV is same as described above. Major countermeasure is breakage of TYLCV infection cycle by, for example, control of whitefly which is, an insect vector for TYLCV, and early removal of infected plants.

An insect proof net with a mesh size of 0.4 mm or less is effective for preventing the entrance of whitefly, but use of such an insect proof net for prevention may cause a temperature elevation inside a greenhouse. Therefore, use of the insect proof net is actually difficult in the cultivation scene.

In addition, major tomato production areas, such as Kyushu region, have various cultivation types with different cultivation term, therefore cultivate tomatoes somewhere throughout a year. Whitefly carrying TYLCV moves between fields and greenhouse in different cultivation types of tomatoes and can survive even during cold winter. Such lack of breakage in the TYLCV infection cycle makes TYLCV prevention difficult.

Recently, an insecticide resistant whitefly, Bemisia tabaci-Biotype Q, is spreading in a lot of region and the insecticides control appears to be limited.

TYLCV tolerant genes such as Ty-1, Ty-2, and Ty-3 have been found in wild relatives tomato. However presence of these genes suppresses disease symptoms, cannot prevent TYLCV infection *per se.*

Tomato varieties introgressed with TYLCV tolerant genes by conventional breeding are already available in the market. However, all varieties are known to be infected by TYLCV and the virus is known to propagate in tomato body. Accordingly, failure to control whitefly during the cultivation of the tomatoes carrying TYLCV tolerant gene results in the tomatoes infected TYLCV Such tomatoes will be a source of transmission TYLCV even when the disease symptoms of TYLCV are suppressed, and cause surrounding TYLCV sensitive tomato varieties to be exposed to the danger of TYLCV infection (see, for example, NPLs 2 to 4).

In view of such circumstances, the present inventors have made intensive studies on the production of TYLCV resistant solanaceous plants by genome editing, and have already identified several genes associated with TYLCV resistance in solanaceous plants (see Patent Literature (hereinafter, abbreviated PTL) 1).

### Citation List

### Patent Literature

PTL 1
International Publication No. WO 2020/111149

### Non-Patent Literature

NPL 1
   European Food Safety Authority, "EFSA Journal," 2013, 11(4):3162
NPL 2
   B. Mabvakure et al., "Virology," 2016, 498: 257-264
NPL 3
   J. Basak, "Journal of Plant Pathology & Microbiology," 2016, 7: 346
NPL 4
   H. Czosnek ed., "Tomato Yellow Leaf Curl Virus Disease", Springer, 2007, pp.85-118

### Summary of Invention

### Technical Problem

Conventional methods for preventing plant viruses mentioned above was difficult to control viruses belonging to the genus *Begomovirus* which cause yellow leaf curl symptom on tomatoes, such as TYLCV. Further, even when solanaceous plant varieties introgressed with TYLCV resistance genes suppressed the symptoms of such viruses, the virus was still able to propagate in the plant body. Therefore, the virus transmission cycle could not be split completely.

Although the present inventors have already disclosed several genes associated with TYLCV resistance in solanaceous plants, there is no specific disclosure on mutations in DCL3 gene (Solyc08g067210), 4CL06 gene (Solyc06g068650), and RLK2 gene (Solyc03g043770)

Under the above-mentioned circumstances, task of the present disclosure is to provide a virus resistant solanaceous plant, a solanaceous plant cell, and a method for producing the solanaceous plant, in which the solanaceous plant has inhibitory properties against: infection by a virus of the genus *Begomovirus* causing tomato yellow leaf curl symptoms (for example TYLCV), propagation of the infected virus, and/or appearance of infection symptoms.

### Solution to Problem

The present disclosure relates to the following virus resistant solanaceous plant, parts of the plant, and processed material thereof.
[1] A solanaceous plant having a mutation in at least one gene selected from a group consisting of DCL3 gene which is silencing associated dicer gene, and a gene homologous thereto; 4CL06 gene which is 4-coumarate-CoA ligase gene, and a gene homologous thereto; and RLK2 gene (Solyc03g043770) which is receptor-like kinase gene, and a gene homologous thereto exclusive of RLK1 gene (Solyc02g091840),
   wherein the mutation either inhibits expression of the mutated gene or makes a protein encoded by the mutated gene to be non-functional for a virus of genus *Begomovirus* causing tomato yellow leaf curl symptoms, and
   wherein the solanaceous plant has virus resistance against the virus.
[2] The solanaceous plant according to [1], wherein the virus of genus *Begomovirus* causing tomato yellow leaf curl symptoms is Tomato yellow leaf curl virus.
[3] The solanaceous plant according to [1] or [2], wherein the mutation is a genomic gene mutation introduced by genome editing techniques.
[4] The solanaceous plant according to any one of [1] to [3], wherein the mutation is in the DCL3 gene which is the silencing associated dicer gene, or the gene homologous thereto.
[5] The solanaceous plant according to any one of [1] to [3], wherein the mutation is in the 4CL06 gene which is the 4-coumarate-CoA ligase gene, or the gene homologous thereto.
[6] The solanaceous plant according to [4] or [5], wherein the mutation is at least one type of mutation selected from (a) to (d) below:
   (a) a frameshift mutation,
   (b) a nonsense mutation,
   (c) a loss of continuous or non-continuous 3n nucleotides (wherein n = 1 to 20), and
   (d) a replacement, deletion, addition, and/or insertion of 1 or more nucleotides.
[7] The solanaceous plant according to [4] or [6], wherein:
   the DCL3 gene which is the silencing associated dicer gene has a cDNA sequence comprising a nucleotide sequence as set forth in SEQ ID NO:1, and
   the gene homologous to the DCL3 gene which is the silencing associated dicer gene has a cDNA sequence comprising a nucleotide sequence which has at least 85% homology to the nucleotide sequence as set forth in SEQ ID NO: 1.
[8] The solanaceous plant according to [7], wherein the DCL3 gene which is the silencing associated dicer gene, or the gene homologous thereto has the mutation in a region corresponding to a nucleotide sequence as set forth in SEQ ID NO:2.
[9] The solanaceous plant according to [8], wherein the mutation is present in a region corresponding to a nucleotide sequence as set forth in SEQ ID NO:3 in the DCL3 gene, which is the silencing associated dicer gene, or the gene homologous thereto.
[10] The solanaceous plant according to [8], wherein the region corresponding to the nucleotide sequence as set forth in SEQ ID NO:2 is mutated to a nucleotide sequence selected from those as set forth in SEQ ID NOs:21 to 26.
[11] The solanaceous plant according to [4] or [7], wherein:
   the 4CL06 gene which is the 4-coumarate-CoA ligase gene has a cDNA sequence comprising a nucleotide sequence as set forth in SEQ ID NO:4, and
   the gene homologous to the 4CL06 gene which is the 4-coumarate-CoA ligase gene has a cDNA sequence comprising a nucleotide sequence which has at least 85% homology to the nucleotide sequence as set forth in SEQ ID NO:4.
[12] The solanaceous plant according to [11], wherein the 4CL06 gene which is the 4-coumarate-CoA ligase gene, or the gene homologous thereto has the mutation in a region corresponding to a nucleotide sequence as set forth in SEQ ID NO:5.
[13] The solanaceous plant according to [12], wherein the mutation is present in a region corresponding to a nucleotide sequence as set forth in SEQ ID NO:6 in the 4CL06 gene which is 4-coumarate-CoA ligase gene, or the gene homologous thereto.
[14] The solanaceous plant according to [12], wherein the region corresponding to the nucleotide sequence as set forth in SEQ ID NO:5 is mutated to a nucleotide sequence selected from those as set forth in SEQ ID NOs:27 and 28.
[15] The solanaceous plant according to any one of [1] to [3], wherein the mutation is in the RLK2 gene (Solyc03g043770) which is the receptor-like kinase gene, or the gene homologous thereto.
[16] The solanaceous plant according to [15], wherein at least one gene selected from a group consisting of the RLK2 gene (Solyc03g043770) which is receptor-like kinase gene, and the gene homologous thereto has a mutation, and the mutation is at least one type of mutation selected from (c) and (d) below:
   (c) a loss of continuous or non-continuous 3n nucleotides (wherein n = 1 to 99), and
   (d) a replacement, deletion, addition, and/or insertion of 1 or more nucleotides.
[17] The solanaceous plant according to [16], wherein the mutation is at least one type of mutation selected from (c) and (d') below:
   (c) a loss of continuous or non-continuous 3n nucleotides (wherein n = 1 to 99), and
   (d') an insertion of continuous or non-continuous 3n nucleotides (wherein n = 1 to 2).
[18] The solanaceous plant according to any one of [15] to [17], wherein:
   the RLK2 gene which is receptor-like kinase gene has a cDNA sequence comprising a nucleotide sequence as set forth in SEQ ID NO:29, and
   the gene homologous to the RLK2 gene which is the receptor-like kinase gene has a cDNA sequence comprising a nucleotide sequence which has at least 85% homology to the nucleotide sequence as set forth in SEQ ID NO:29.
[19] The solanaceous plant according to [18], wherein the RLK2 gene which is the receptor-like kinase gene or the gene homologous thereto has the mutation in a region corresponding to a nucleotide sequence as set forth in SEQ ID NO:30.
[20] The solanaceous plant according to [19], wherein the mutation is present in a region corresponding to a nucleotide sequence as set forth in SEQ ID NO:36.
[21] The solanaceous plant according to [19] or [20], wherein the region corresponding to the nucleotide sequence as set forth in SEQ ID NO:30 is mutated to a nucleotide sequence selected from those as set forth in SEQ ID NOs:34, 35 and 40.
[22] The solanaceous plant according to any one of [1] to [21] which is a tomato.
[23] A part of the solanaceous plant according to any one of [1] to [22].
[24] The part of the solanaceous plant according to [23] which is a fruit.
[25] The part of the solanaceous plant according to [23] which is a seed.
[26] A processed material of the solanaceous plant or the part thereof according to any one of [1] to [22].
[27] The processed material according to [26] which is edible.

Further, the present disclosure relates to the following solanaceous plant cell, a plant and a part thereof comprising the cell, and processed material thereof.

A solanaceous plant cell having a mutation in at least one gene selected from a group consisting of DCL3 gene which is silencing associated dicer gene, and a gene homologous thereto; 4CL06 gene which is 4-coumarate-CoA ligase gene, and a gene homologous thereto; and RLK2 gene (Solyc03g043770) which is receptor-like kinase gene, and a gene homologous thereto exclusive of RLK1 gene (Solyc02g091840),
wherein the mutation either inhibits expression of the mutated gene or makes a protein encoded by the mutated gene to be non-functional for a virus of genus *Begomovirus* causing tomato yellow leaf curl symptoms, and
wherein the solanaceous plant cell has virus resistance against the virus.

The solanaceous plant cell according to [28], wherein the virus of genus *Begomovirus* causing tomato yellow leaf curl symptoms is Tomato yellow leaf curl virus.

The solanaceous plant cell according to [28] or [29], wherein the mutation is a genomic gene mutation introduced by genome editing techniques.

The solanaceous plant cell according to any one of [28] to [30], wherein the mutation is in the DCL3 gene which is the silencing associated dicer gene, or the gene homologous thereto.

The solanaceous plant cell according to any one of [28] to [30], wherein the mutation is in the 4CL06 gene which is the 4-coumarate-CoA ligase gene, or the gene homologous thereto.

The solanaceous plant cell according to [31] or [32], wherein the mutation is at least one type of mutation selected from (a) to (d) below:
(a) a frameshift mutation,
(b) a nonsense mutation,
(c) a loss of continuous or non-continuous 3n nucleotides (wherein n = 1 to 20), and
(d) a replacement, deletion, addition, and/or insertion of 1 or more nucleotides.

The solanaceous plant cell according to [31] or [33], wherein:
the DCL3 gene which is the silencing associated dicer gene has a cDNA sequence comprising a nucleotide sequence as set forth in SEQ ID NO:1, and
the gene homologous to the DCL3 gene which is the silencing associated dicer gene has a cDNA sequence comprising a nucleotide sequence which has at least 85% homology to the nucleotide sequence as set forth in SEQ ID NO:1.

The solanaceous plant cell according to [34], wherein the DCL3 gene which is the silencing associated dicer gene, or the gene homologous thereto has the mutation in a region corresponding to a nucleotide sequence as set forth in SEQ ID NO:2.

The solanaceous plant cell according to [35], wherein the mutation is present in a region corresponding to a nucleotide sequence as set forth in SEQ ID NO:3 in the DCL3 gene, which is the silencing associated dicer gene, or the gene homologous thereto.

The solanaceous plant cell according to [35], wherein the region corresponding to the nucleotide sequence as set forth in SEQ ID NO:2 is mutated to a nucleotide sequence selected from those as set forth in SEQ ID NOs:21 to 26.

The solanaceous plant cell according to [31] or [34], wherein:
the 4CL06 gene which is the 4-coumarate-CoA ligase gene has a cDNA sequence comprising a nucleotide sequence as set forth in SEQ ID NO:4, and
the gene homologous to the 4CL06 gene which is the 4-coumarate-CoA ligase gene has a cDNA sequence comprising a nucleotide sequence which has at least 85% homology to the nucleotide sequence as set forth in SEQ ID NO:4.

The solanaceous plant cell according to [38], wherein the 4CL06 gene which is the 4-coumarate-CoA ligase gene, or the gene homologous thereto has the mutation in a region corresponding to a nucleotide sequence as set forth in SEQ ID NO:5.

The solanaceous plant cell according to [39], wherein the mutation is present in a region corresponding to a nucleotide sequence as set forth in SEQ ID NO:6 in the 4CL06 gene which is 4-coumarate-CoA ligase gene, or the gene homologous thereto.

The solanaceous plant cell according to [39], wherein the region corresponding to the nucleotide sequence as set forth in SEQ ID NO:5 is mutated to a nucleotide sequence selected from those as set forth in SEQ ID NOs:27 and 28.

The solanaceous plant cell according to any one of [28] to [30], wherein the mutation is in the RLK2 gene (Solyc03g043770) which is the receptor-like kinase gene, or the gene homologous thereto.

The solanaceous plant cell according to [42], wherein at least one gene selected from a group consisting of the RLK2 gene (Solyc03g043770) which is receptor-like kinase gene, and the gene homologous thereto has a mutation, and the mutation is at least one type of mutation selected from (c) and (d) below:
(c) a loss of continuous or non-continuous 3n nucleotides (wherein n = 1 to 99), and
(d) a replacement, deletion, addition, and/or insertion of 1 or more nucleotides.

The solanaceous plant cell according to [43], wherein the mutation is at least one type of mutation selected from (c) and (d') below:
(c) a loss of continuous or non-continuous 3n nucleotides (wherein n = 1 to 99), and
(d') an insertion of continuous or non-continuous 3n nucleotides (wherein n = 1 to 2).

The solanaceous plant cell according to any one of [42] to [44], wherein:
the RLK2 gene which is receptor-like kinase gene has a cDNA sequence comprising a nucleotide sequence as set forth in SEQ ID NO:29, and
the gene homologous to the RLK2 gene which is the receptor-like kinase gene has a cDNA sequence comprising a nucleotide sequence which has at least 85% homology to the nucleotide sequence as set forth in SEQ ID NO:29.

The solanaceous plant cell according to [45], wherein the RLK2 gene which is the receptor-like kinase gene or the gene homologous thereto has the mutation in a region corresponding to a nucleotide sequence as set forth in SEQ ID NO:30.

The solanaceous plant cell according to [46], wherein the mutation is present in a region corresponding to a nucleotide sequence as set forth in SEQ ID NO:36.

The solanaceous plant cell according to [46] or [47], wherein the region corresponding to the nucleotide sequence as set forth in SEQ ID NO:30 is mutated to a nucleotide sequence selected from those as set forth in SEQ ID NOs:34, 35 and 40.

The solanaceous plant cell according to any one of [28] to [48], wherein the solanaceous plant is a tomato.

A solanaceous plant and a part thereof comprising the solanaceous plant cell according to any one of [28] to [49], and having virus resistance against a virus of genus *Begomovirus* causing tomato yellow leaf curl symptoms.

The part of the solanaceous plant according to [50] which is a fruit.

The part of the solanaceous plant according to [50] which is a seed.

A processed material of the solanaceous plant or the part thereof according to any one of [50] to [52].

The processed material according to [53] which is edible.

Further the present disclosure provides the following method for producing a solanaceous plant and a solanaceous plant produced by the method.

A method for producing a virus resistant solanaceous plant which is resistant to a virus of genus *Begomovirus* causing tomato yellow leaf curl symptoms, the method comprising:
selecting at least one gene from a group consisting of DCL3 gene which is silencing associated dicer gene, and a gene homologous thereto; 4CL06 gene which is 4-coumarate-CoA ligase gene, and a gene homologous thereto; and RLK2 gene (Solyc03g043770) which is receptor-like kinase gene, and a gene homologous thereto exclusive of RLK1 gene (Solyc02g091840),
introducing a mutation into the selected gene in a genome, wherein the introduced mutation is either a mutation inhibiting an expression of the mutated gene or a mutation making a protein encoded by the mutated gene to be non-functional for a virus of genus *Begomovirus* causing tomato yellow leaf curl symptoms; and
selecting a solanaceous plant having resistance to the virus.

The method for producing a virus resistant solanaceous plant according to [55], wherein the virus of genus *Begomovirus* causing tomato yellow leaf curl symptoms is Tomato yellow leaf curl virus.

The method for producing a virus resistant solanaceous plant according to [55] or [56], wherein the mutation is introduced into the gene in the genome by genome editing techniques.

The method for producing a virus resistant solanaceous plant according to any one of [55] to [57], wherein the mutation is introduced into the DCL3 gene which is the silencing associated dicer gene, or the gene homologous thereto.

The method for producing a virus resistant solanaceous plant according to any one of [55] to [57], wherein the mutation is introduced into the 4CL06 gene which is the 4-coumarate-CoA ligase gene, or the gene homologous thereto.

The method for producing a virus resistant solanaceous plant according to [58] or [59], wherein the mutation is at least one type of mutation selected from (a) to (d) below:
(a) a frameshift mutation,
(b) a nonsense mutation,
(c) a loss of continuous or non-continuous 3n nucleotides(wherein n = 1 to 20), and
(d) a replacement, deletion, addition, and/or insertion of 1 or more nucleotides.

The method for producing a virus resistant solanaceous plant according to [58] or [60], wherein:
the DCL3 gene which is the silencing associated dicer gene has a cDNA sequence comprising a nucleotide sequence as set forth in SEQ ID NO:1, and
the gene homologous to the DCL3 gene which is the silencing associated dicer gene has a cDNA sequence comprising a nucleotide sequence which has at least 85% homology to the nucleotide sequence as set forth in SEQ ID NO:1.

The method for producing a virus resistant solanaceous plant according to [61], wherein the mutation is introduced into the DCL3 gene which is the silencing associated dicer gene, or the gene homologous thereto at a region corresponding to a nucleotide sequence as set forth in SEQ ID NO:2.

The method for producing a virus resistant solanaceous plant according to [62], wherein the mutation is introduced into a region corresponding to a nucleotide sequence as set forth in SEQ ID NO:3 in the DCL3 gene which is the silencing associated dicer gene, or the gene homologous thereto.

The method for producing a virus resistant solanaceous plant according to [62], wherein the mutation is introduced so that the region corresponding to the nucleotide sequence as set forth in SEQ ID NO:2 is changed to a nucleotide sequence selected from those as set forth in SEQ ID NOs:21 to 26.

The method for producing a virus resistant solanaceous plant according to [59] or [60], wherein the 4CL06 gene which is the 4-coumarate-CoA ligase gene has a cDNA sequence comprising a nucleotide sequence as set forth in SEQ ID NO:4, and
the gene homologous to the 4CL06 gene which is the 4-coumarate-CoA ligase gene has a cDNA sequence comprising a nucleotide sequence which has at least 85% homology to the nucleotide sequence as set forth in SEQ ID NO:4.

The method for producing a virus resistant solanaceous plant according to [65], wherein the mutation is introduced into a region corresponding to a nucleotide sequence as set forth in SEQ ID NO:5 in the 4CL06 gene which is the 4-coumarate-CoA ligase gene, or the gene homologous thereto.

The method for producing a virus resistant solanaceous plant according to [66], wherein the mutation is introduced into a region corresponding to a nucleotide sequence as set forth in SEQ ID NO:6 in the 4CL06 gene which is the 4-coumarate-CoA ligase gene, or the gene homologous thereto.

The method for producing a virus resistant solanaceous plant according to [66], wherein the mutation is introduced so that the region corresponding to the nucleotide sequence as set forth in SEQ ID NO:5 is changed to a nucleotide sequence set forth in SEQ ID NO:27 or 28.

The method for producing a virus resistant solanaceous plant according to any one of [55] to [57], wherein the mutation is introduced into the RLK2 gene (Solyc03g043770) which is receptor-like kinase gene, and a gene homologous thereto.

The method for producing a virus resistant solanaceous plant according to [69], wherein the mutation is at least one type of mutation selected from (c) and (d) below:
(c) a loss of continuous or non-continuous 3n nucleotides (wherein n = 1 to 99), and
(d) a replacement, deletion, addition, and/or insertion of 1 or more nucleotides.

The method for producing a virus resistant solanaceous plant according to [70], wherein the mutation is at least one type of mutation selected from (c) and (d') below:
(c) a loss of continuous or non-continuous 3n nucleotides (wherein n = 1 to 99), and
(d') an insertion of continuous or non-continuous 3n nucleotides (wherein n = 1 to 2).

The method for producing a virus resistant solanaceous plant according to any one of [69] to [71], wherein:
the RLK2 gene which is receptor-like kinase gene has a cDNA sequence comprising a nucleotide sequence as set forth in SEQ ID NO:29, and
the gene homologous to the RLK2 gene which is the receptor-like kinase gene has a cDNA sequence comprising a nucleotide sequence which has at least 85% homology to the nucleotide sequence as set forth in SEQ ID NO:29.

The method for producing a virus resistant solanaceous plant according to [72], wherein the mutation is introduced into the RLK2 gene which is receptor-like kinase gene, and a gene homologous thereto at a region corresponding to a nucleotide sequence as set forth in SEQ ID NO:30.

The method for producing a virus resistant solanaceous plant according to [73], wherein the mutation is introduced into a region corresponding to a nucleotide sequence as set forth in SEQ ID NO:36.

The method for producing a virus resistant solanaceous plant according to [73] or [74], wherein the mutation is introduced so that the region corresponding to the nucleotide sequence as set forth in SEQ ID NO:30 is changed to a nucleotide sequence as set forth in SEQ ID NOs:34, 35 or 40.

The method for producing a virus resistant solanaceous plant according to any one of [55] to [75], wherein the solanaceous plant is a tomato.

A virus resistant solanaceous plant obtained by the production method according to any one of [55] to [76].

In addition, the present disclosure provides the following method for producing a bred progeny of a solanaceous plant and a solanaceous plant obtained by the production method.

A method for producing a bred progeny of a virus resistant solanaceous plant which is resistant to a virus of genus *Begomovirus* which causes tomato yellow leaf curl symptoms, the method comprising: self-pollination or cross-pollination of either a virus resistant solanaceous plant obtained by the production method according to any one of [55] to [76] or a progeny of the virus resistant solanaceous plant.

A virus resistant solanaceous plant resistant to a virus of genus *Begomovirus* which causes tomato yellow leaf curl symptoms, the solanaceous plant being obtained by the production method of [78].

### Advantageous Effects of Invention

According to the present disclosure, there is provided a virus resistant solanaceous plant, a solanaceous plant cell, and a method for producing the solanaceous plant, in which the solanaceous plant has inhibitory properties against: infection by a virus of genus *Begomovirus* causing tomato yellow leaf curl symptoms, propagation of the infected virus, and/or expression of infection symptoms.

### Brief Description of Drawings

FIG. 1 is a cDNA sequence of tomato silencing-associated Dicer gene (DCL3 gene) in which a single underlined portion (1089th nucleotide to 1348th nucleotide) is exon 8, a double underlined portion is a guide RNA recognition site (1308th nucleotide to 1327th nucleotide), and a portion enclosed in box is PAM sequence;
FIG. 2 is a cDNA sequence of tomato 4-coumarate-CoA ligase gene (4CL06 gene) on chromosome 6 in which a single underlined portion (669th nucleotide to 1136th nucleotide)is exon 2, a double underlined portion is a guide RNA recognition site (954th nucleotide to 973rd nucleotide), and a portion enclosed in box is PAM sequence;
FIG. 3a shows a tomato plant without any symptoms of TYLCV infection;
FIG. 3b shows a tomato plant showing symptoms of TYLCV infection;
FIG. 4 is a graph showing morbidities for T1 seedlings of DCL3 gene-edited line and control tomato plants free of introduced mutation, the morbidities being calculated based on PCR analysis on 21 days (first test) or 27 days (second test) post viral inoculation; and in the drawing, dpi represents the number of days post inoculation, Cont represents a control, and DCL3 represents DCL3 gene-edited line;
FIG. 5 is a result of PCR analysis of the T1 plants of the DCL3 gene-edited line in which the PCR products have been subjected to agarose gel electrophoresis, and in the drawing, Cont represents a control, and DCL3 represents DCL3 gene-edited line;
FIG. 6 is a graph showing morbidities for T1 seedlings of 4CL06 gene-edited line G27 and control tomato plants free of introduced mutation, the morbidities being calculated based on PCR analysis, on 21 (1st test) or day 24 (2nd test) days post viral inoculation, and in the drawing, dpi represents the number of days post inoculation, Cont represents a control, and G27 represents 4CL06 gene-edited line G27;
FIG. 7 is a result of PCR analysis of the T1 plants of the 4CL06 gene-edited line in which the PCR products have been subjected to agarose gel electrophoresis, and in the drawing, Cont represents a control, and G27 represents 4CL06 gene-edited line G27;
FIG. 8 is a drawing showing the mutation patterns of tomato DCL3 gene; in wild type nucleotide sequence, a double underlined portion is a guide RNA recognition site, and a portion enclosed in box is PAM sequence; and in mutated nucleotide sequence, "•" represents an absence (deletion) of a nucleotide as compared to the wild type DCL3 gene, and single underlined nucleotide represents a replacement as compared to the wild type DCL3 gene.
FIG. 9 is a drawing showing the mutation patterns of tomato 4CL06 gene; in wild type nucleotide sequence, a double underlined portion is a guide RNA recognition site, and a portion enclosed in box is PAM sequence; and in mutated nucleotide sequence, "•" represents an absence (deletion) of a nucleotide as compared to the wild type 4CL06 gene.
FIG. 10 is a cDNA sequence of tomato receptor-like kinase gene (RLK2 gene) on chromosome 3 in which an underlined portion (1st nucleotide to 830th nucleotide) is exon 1, a wavy underlined portion is a guide RNA recognition site (277th nucleotide to 296th nucleotide), and a portion enclosed in box is PAM sequence;
FIG. 11 is a graph showing infection rates for T1 seedlings of P15 line, an RLK2 mutant line, and control tomato seedlings free of introduced mutation, the infection rates being calculated based on PCR analysis on 28 days post viral inoculation;
FIG. 12 is an electropherogram showing the results of RT-PCR analysis of the 2nd TSWV inoculation test;
FIG. 13 is a drawing showing mutation patterns in the tomato receptor-like kinase RLK2 gene, in which a broken line represents deletion and a nucleotide in a box represents replacement;
FIG. 14 is a schematic drawing showing the expected motifs and their positions in the amino acid sequence of the tomato receptor-like kinase RLK2;
FIG. 15 is a graph showing infection rates for T1 seedlings of P24 line, an RLK2 mutant line, and control tomato seedlings free of introduced mutation, the infection rates being calculated based on PCR analysis on 28 days post viral inoculation;
FIG. 16 is an electropherogram showing the results of RT-PCR analysis of TSWV inoculation test, in which P and N in the drawing represent positive and negative controls, respectively;
FIG. 17 is a graph showing infection rates for T1 seedlings of P19 line, an RLK2 mutant line, and control tomato seedlings free of introduced mutation, the infection rates being calculated based on PCR analysis on 28 days post viral inoculation;
FIG. 18 is a graph showing infection rates for T1 seedlings of DC3-2D line, an DCL3 mutant line, and control tomato seedlings free of introduced mutation, the infection rates being calculated based on PCR analysis on 28 days post viral inoculation;
FIG. 19a is an electropherogram showing the results of RT-PCR analysis of TSWV inoculation test for DC3-2D line, and N in the drawing represents negative control; and
FIG. 19b is an electropherogram showing the results of RT-PCR analysis of TSWV inoculation test for control plant, and N in the drawing represents negative control.

### Description of Embodiments

Present inventors have conducted extensive and intensive studies for solving the above-mentioned problems, and found that, when solanaceous plants have a mutation in silencing-associated Dicer gene (hereinbelow, frequently referred to also as DCL3 gene) or a gene homologous thereto, and/or 4-coumarate-CoA ligase gene on chromosome 6 (hereinbelow, frequently referred to also as 4CL06 gene) or a gene homologous thereto, and/or receptor-like kinase RLK gene on chromosome 3 (hereinbelow, frequently referred to also as "RLK2 gene" or "BAM2 gene") or a gene homologous thereto, and the mutation either inhibits expression of the mutated gene (the DCL3 gene, 4CL06 gene, RLK2 gene, or genes homologous thereto) or makes a protein encoded by the mutated gene to be non-functional for a virus of genus *Begomovirus* causing tomato yellow leaf curl symptoms (e.g., TYLCV), TYLCV infection rate decreases and the solanaceous plants have virus resistance against the virus. This is a first report on a virus resistant plant having mutated DCL3 gene, 4CL06 gene, or RLK2 gene in solanaceous plants.

Embodiments of the present disclosure (hereinafter, may be referred to as "present embodiment") are explained in detail below. The present disclosure is not limited to the present embodiments and the drawings, and may be practiced with various changes within the scope of the gist of the present disclosure

In the present disclosure, there is no specific limitation with respect to the virus of genus *Begomovirus* causing tomato yellow leaf curl symptoms, but the virus is preferably Tomato yellow leaf curl virus (TYLCV). Hereinbelow, the present disclosure is explained using TYLCV as a specific example of the virus of the genus *Begomovirus* causing tomato yellow leaf curl symptoms, but the explanation should not be construed as limiting the virus mentioned in the present disclosure to TYLCV. It could be understood that the term "TYLCV" in the explanation below may be read as "a virus of the genus *Begomovirus* causing yellow leaf curl symptoms in solanaceous plants."

### [I] Virus resistant solanaceous plant

In one aspect, the present embodiment relates to a virus (e.g., TYLCV) resistant solanaceous plant. In the present embodiment, the virus resistant solanaceous plant is, for example, a plant having the properties of inhibiting the infection by a virus causing yellow leaf curl symptoms in tomato (e.g., TYLCV), suppressing the propagation of the virus when infected, and/or suppressing the expression of the virus infection symptoms. The virus resistant solanaceous plant is preferably a plant having a property of inhibiting the virus infection, or when infected, inhibiting the propagation of the virus.

In the present embodiment, the "Tomato yellow leaf curl virus (TYLCV)" refers to viruses classified under the family Geminiviridae and genus *Begomovirus* , which have a circular single DNA as a monopartite genome and a geminate virions that each having a diameter of about 20 nm.

TYLCV is mainly occurring in Middle East, North and Central America, Southeast Asia, East Asia (Japan, Korea and China) and the like. There are two TYLCV strains occurring in Japan: TYLCV Israel strain which includes an isolate found in Nagasaki and which is occurring in Kyushu area, Kanto area, etc., and Israel-mild strain which is occurring in Tokai area, Kanto area, etc.

In the present embodiment, there is no particular limitation with respect to the solanaceous plants as long as the plants belongs to the family Solanaceae, and such plants include those belonging to the genus *Solanum,* genus *Nicotiana,* genus *Capsicum* or the like. Specific examples of such plants include tomato (*Solanum lycopersicum*)*,* eggplant (*Solanum melongena*), tobacco (*Nicotiana tabacum*)*,* hot pepper (*Capsicum annuum*)*,* potato (*Solanum tuberosum*) and the like, and the plants are preferably tomato, eggplant or potato, and more preferably tomato.

In one aspect, the TYLCV resistant solanaceous plant of the present embodiment has a mutation in at least one gene selected from a group consisting of silencing associated dicer gene (DCL3 gene; Solyc08g067210) and a gene homologous thereto, 4-coumarate-CoAligase gene on chromosome 6 (4CL06 gene; Solyc06g068650) and a gene homologous thereto, and receptor-like kinase gene on chromosome 3 (RLK2 gene or BAM2 gene; Solyc03g043770) and a gene homologous thereto.

### (DCL3 gene)

DCL3 gene is a type of enzyme called Dicer. Four types of Dicer (DCL1 to DCL4) are known to exist in plants. Among these, DCL3 preferentially cleave short double stranded RNA of 50 nucleotides or less and produces an RNA of 24 nucleotides long. This 24-nucleotide short RNA (siRNA) is known to participate in deactivation of 24-nucleotide transposon functioning in DNA methylation, and maintenance of heterochromatin (H. Nagano et al., "Nucleic Acid Research," 2014, 42(3): 1845-1856).

DCL3 gene is known to exist in various plants, such as *Arabidopsis thaliana* and tomato. Genes homologous to tomato DCL3 gene are also known to exist in other solanaceous plants. For example, gene called DCL3AEndoribonuclease Dicer homolog 3a which is a gene homologous to tomato DCL3 exists in eggplants.

All of such homologous genes of DCL3 which are known for solanaceous plants other than tomatoes are included in the homologous genes of DCL3 gene in accordance with the present disclosure.

In the present embodiment, the "DCL3 gene" is preferably a gene having a cDNA sequence which either comprises the nucleotide sequence as set forth in SEQ ID NO:1 or consists of the nucleotide sequence as set forth in SEQ ID NO:1. Herein, the "cDNA sequence" is a DNA sequence synthesized by reverse transcription from an mRNA transcribed from a gene, and is a DNA sequence without introns found in the gene and consisting only of protein coding regions.

In addition, in the present embodiment, the "gene homologous to the DCL3 gene" is preferably a gene having a cDNA sequence which either comprises a nucleotide sequence having sequence homology to the nucleotide sequence as set forth in SEQ ID NO:1, or consists of a nucleotide sequence having sequence homology to the nucleotide sequence as set forth in SEQ ID NO: 1. In the present disclosure, "sequence homology" means sequence identity. There is no particular limitation on the degree of sequence homology with the nucleotide sequence as set forth in SEQ ID NO:1, but the sequence homology is preferably at least 85% and less than 100%. Minimum sequence homology may be any value, such as at least 87%, at least 90%, at least 93%, at least 95%, at least 97%, at least 99%, and at least 99.5%. Homology between the nucleotide sequence as set forth in SEQ ID NO:1 and the cDNA sequence of the homologous gene may be determined by conventional methods. For example, homology between nucleotide sequences may be determined using conventional homology search programs, such as BLAST.

### (4CL06 gene)

4-coumarate-CoA ligase is one of ligases and mainly forms a carbon-sulfur linkage. There are several isoforms of the 4-coumarate-CoA ligase which are encoded by a small gene family, and which catalyze a reaction where hydroxy or methoxy cinnamic acid derivatives are converted into corresponding thioesters. Such thioesters are associated with biosynthesis of phenyl propanoids known to have many nutritional and medical utilities. On the other hand, relation between 4-coumarate-CoA ligase gene (4CL06 gene) and viruses is unknown. Number of reports in relation to Begomovirus infection is very small and involvement is unknown, but involvement of 4CL06 gene in other biological stress, such as infection of Alternaria bacteria, or non-biological stress, such as drought and salt sensitivity have been reported (S.G. Lavhale et al., "Planta," 2018, 248: 1063-1078).

In the present embodiment, the "4CL06 gene" is preferably a gene having a cDNA sequence which either comprises a nucleotide sequence as set forth in SEQ ID NO:4, or consists of the nucleotide sequence as set forth in SEQ ID NO:4.

In the present embodiment, the "gene homologous to the 4CL06 gene" is preferably a gene having a cDNA sequence which either comprises a nucleotide sequence which has sequence homology to the nucleotide sequence as set forth in SEQ ID NO:4, or consists of a nucleotide sequence which has sequence homology to the nucleotide sequence as set forth in SEQ ID NO:4. In the present disclosure, "sequence homology" means sequence identity. There is no particular limitation on the degree of sequence homology with the nucleotide sequence as set forth in SEQ ID NO:4, but the sequence homology is preferably at least 85% and less than 100%. Minimum sequence homology may be any value, such as at least 87%, at least 90%, at least 93%, at least 95%, at least 97%, at least 99%, and at least 99.5%. Homology between the nucleotide sequence as set forth in SEQ ID NO:4 and the cDNA sequence of the homologous gene may be determined by conventional methods. For example, homology between nucleotide sequences may be determined using conventional homology search programs, such as BLAST.

### (RLK2 gene)

The RLK gene is a tomato gene encoding "Receptor-Like Kinase," that is a kinase resembling a receptor. RLK is called BAM1 (Barely Any Meristem 1) in *Arabidopsis thaliana* and the gene encodes CLAVATA1 related receptor-like kinase protein necessary for meristematic functions of shoots and flowers which are related to the formation of leaves and gametes. Further, in *Arabidopsis thaliana,* presence of BAM2 which is highly homologous to BAM1 has been recognized, and from recent studies, presence of a highly homologous homologue in tomato is beginning to be understood. "RLK1 gene" (Solyc02g091840 on chromosome 2) and "RLK2 gene" (Solyc03g043770 on chromosome 3) of tomato are known as such homologues, and the present disclosure relates to the RLK2 gene. Regarding BAM1 and BAM2 of *Arabidopsis thaliana,* although relationship with C4 protein of a close relative to TYLCV, gene silencing and involvement in viral transfer have been suggested, specific functions of the genes have not been elucidated.

In the present embodiment, the "RLK2 gene" is preferably a gene having a cDNA sequence which either comprises the nucleotide sequence as set forth in SEQ ID NO:29, or consists of the nucleotide sequence as set forth in SEQ ID NO:29.

In the present embodiment, the "gene homologous to the RLK2 gene" is preferably a gene having a cDNA sequence which either comprises a nucleotide sequence which has sequence homology to the nucleotide sequence as set forth in SEQ ID NO:29, or consists of a nucleotide sequence which has sequence homology to the nucleotide sequence as set forth in SEQ ID NO:29. In the present disclosure, "sequence homology" means sequence identity. There is no particular limitation on the degree of sequence homology with the nucleotide sequence as set forth in SEQ ID NO:29, but the sequence homology is preferably at least 85% and less than 100%. Minimum sequence homology may be any value, such as at least 87%, at least 90%, at least 93%, at least 95%, at least 97%, at least 99%, and at least 99.5%. Homology between the nucleotide sequence as set forth in SEQ ID NO:29 and the cDNA sequence of the homologous gene may be determined by conventional methods. For example, homology between nucleotide sequences may be determined using conventional homology search programs, such as BLAST.

Tomato "RLK1 gene" (Solyc02g091840) and the "RLK2 gene" (Solyc03g043770) of the present disclosure are homologous gene of each other, but according to the present disclosure, the gene homologous to the RLK2 gene is exclusive of "RLK1 gene" (Solyc02g091840).

### (TYLCV resistance gene)

According to the present embodiment, the solanaceous plant has a mutation in at least one gene selected from a group consisting of DCL3 gene or a gene homologous thereto, 4CL06 gene or a gene homologous thereto, and RLK2 gene or a gene homologous thereto (hereinafter, the gene having a mutation is also called "virus resistance gene" or "TYLCV resistance gene"). The mutation either inhibits expression of the mutated gene or makes a protein encoded by the mutated gene to be non-functional for a virus of genus *Begomovirus* causing tomato yellow leaf curl symptoms (e.g., TYLCV). The protein which is non-functional for the virus refers to either a protein which cannot be used by the virus during its infection and replication, or a protein which reduces the infection and replication of the virus. In one aspect, the virus (TYLCV) resistance gene may be a gene which has been mutated to no longer encode a protein. Hereinbelow, as a matter of convenience, the present disclosure will be explained by using TYLCV as an example, but the virus (TYLCV) resistance gene of the present disclosure is not limited to a gene resistance to TYLCV.

Although not bound by any theory, during plant infection, TYLCV is considered to use a specific 4-coumarate-CoA ligase isoform among the plurality of 4-coumarate-CoA ligase isoforms present in a solanaceous plant. When a gene encoding the specific isoform used by TYLCV (i.e., 4-coumarate-CoA ligase functional for TYLCV) has a mutation, and the mutation either prevents the production of the specific 4-coumarate-CoA ligase protein used by the TYLCV or causes the produced 4-coumarate-CoA ligase protein to be non-functional for TYLCV, progression of translation of proteins necessary for virus infection and propagation which are encoded by the viral genome is likely to be blocked. Alternatively, the infection and propagation of TYLCV may be inhibited due to incomplete function of a TYLCV protein which needs an interaction with the 4-coumarate-CoA ligase protein. Solanaceous plants are considered to acquire TYLCV resistance in these manner.

Further, although not bound by any theory, during plant infection, TYLCV is considered to use a specific receptor-like kinase isoform among the plurality of receptor-like kinase isoforms present in a solanaceous plant. When a gene encoding the specific isoform used by TYLCV (i.e., receptor-like kinase functional for TYLCV) has a mutation, and the mutation either prevents the production of the specific receptor-like kinase protein used by the TYLCV or causes the produced receptor-like kinase protein to be non-functional for TYLCV, progression of translation of proteins necessary for virus infection and propagation which are encoded by the viral genome is likely to be blocked. Alternatively, the infection and propagation of TYLCV may be inhibited due to incomplete function of a viral protein which needs an interaction with the receptor-like kinase protein. Consequently, Solanaceous plants are considered to acquire TYLCV resistance.

On the other hand, even when one of the multiple 4-coumarate-CoA ligase homologues or receptor-like kinase homologues present in the solanaceous plant becomes mutated, either the plant itself is capable of using other homologues. Alternatively, the plant itself is capable of using the 4-coumarate-CoA ligase homologues or receptor-like kinase homologues non-functional for TYLCV. Accordingly, TYLCV resistance can be given to the plant without causing no or minimum amount of adverse effects on the growth of host solanaceous plant.

Regarding DCL3 gene, as explained above, the presence of 4 types of Dicer (DCL1 to DCL4) are known, and the Dicers are considered to exist in plants while assisting each other.

As explained above, the solanaceous plants having the TYLCV resistance gene acquire TYLCV resistance. For example, a plant may be judged as having the "TYLCV resistance" when the amount of accumulated TYLCV in a plant body is the same or less than the amount in a plant without TYLCV inoculation on 20 or more days post TYLCV inoculation, and/or when symptoms of TYLCV infection cannot be observed visually on 20 or more days post TYLCV inoculation. Specifically, as shown in the below-mentioned Examples, TYLCV resistance of plants may be judged by: infecting plants with TYLCV using a routine method, and detecting accumulation of TYLCV in plant bodies by conventional methods, such as ELISA, PCR, and the like. In addition, TYLCV resistance of plants may be judged by observing the presence or absence of TYLCV symptoms (yellowing, curling (curving), dwarf, chlorosis, etc. of the leaves) (for example, see FIGS. 3a and 3b) in the TYLCV infected plants.

As long as the solanaceous plants have the above-mentioned TYLCV resistance, the gene mutation may be present in at least one gene selected from the group consisting of the DCL3 gene and a gene homologous thereto, 4CL06 gene and a gene homologous thereto, and RLK2 gene and a gene homologous thereto.

Further, when the TYLCV resistant solanaceous plants of the present embodiment have a mutation in the DCL3 gene, the plants may have mutations in all of the genes each encoding the DCL3 protein which is functional for TYLCV. For example, in the case of diploid plants, such as amphidiploid plants, each of the plurality of genes encoding the DCL3 protein functional for TYLCV preferably has a mutation therein. The TYLCV resistant solanaceous plants may have other normal DCL3 gene(s) as long as the TYLCV resistant solanaceous plants have a mutation in the gene encoding the DCL3 protein functional for TYLCV. Further, the TYLCV resistant solanaceous plants may be plants in which endogenous gene(s) encoding the DCL3 protein functional for TYLCV have been made non-functional due to complete loss, damage or the like, but instead, containing an introduced exogenous DCL3 gene.

The same applies to the case where a TYLCV resistant solanaceous plants have the mutation in the 4CL06 gene. In other words, all of the genes encoding any one of the proteins which are functional for TYLCV may have the mutation and, preferably, all of the genes encoding the proteins functional for TYLCV are mutated to TYLCV resistance gene. The TYLCV resistant solanaceous plants may have other normal gene(s), as long as such TYLCV resistant solanaceous plants have a mutation in a gene encoding any one of the proteins which are functional for TYLCV. Further, the TYLCV resistant solanaceous plants may be plants in which the endogenous gene(s) encoding the protein functional for TYLCV have been made unfunctional due to complete loss, damage or the like, but instead, containing an introduced exogenous homologous gene(s).

The same applies to the case where a TYLCV resistant solanaceous plants have the mutation in the RLK2 gene. In other words, the TYLCV resistant solanaceous plants may have other normal gene(s) as long as a mutation is in a gene encoding any one of the proteins which are functional for TYLCV. Further, the TYLCV resistant solanaceous plants may be plants in which the endogenous gene(s) encoding the protein (RLK herein) functional for TYLCV have been made unfunctional due to complete loss, damage or the like, but instead, containing an introduced exogenous homologous gene(s).

In one aspect of the present embodiment, the TYLCV resistant solanaceous plants have a mutation in their genomic gene. There is no particular limitation as long as the gene mutation imparts virus resistance to the plants.

Specific examples of mutation in DCL3 gene and gene homologous thereto, and/or 4CL06 gene and gene homologous thereto include mutations (a) to (d) below:
(a) a frameshift mutation,
(b) a nonsense mutation,
(c) a loss of continuous or non-continuous 3n nucleotides (wherein n = 1 to 20), and
(d) a replacement, deletion, addition, and/or insertion of 1 or more nucleotides.

(a) The frameshift mutation is a mutation where a loss or addition of a nucleotide causes a shift in a reading frame of a codon and the mutated gene encodes a different amino acid sequence. Due to the change in the encoded amino acid sequence, the mutated gene becomes a TYLCV resistance gene.
(b) The nonsense mutation is a mutation where a codon intrinsically encoding an amino acid is changed to a termination codon, and due to this change, the mutated gene becomes a TYLCV resistance gene.
(c) The loss of continuous or non-continuous 3n nucleotides (wherein n = 1 to 20, preferably n = 1 to 10, more preferably n = 1 to 3, for example, the 3n nucleotides are 3, 6 or 9 nucleotides) results in a change in the amino acid sequence encoded downstream of this lost region. Due to the occurrence of such a change, the mutated gene becomes a TYLCV resistance gene.
(d) The replacement, deletion, addition, and/or insertion of 1 or more nucleotides results in a change in a reading frame of an amino acid sequence encoded by a nucleotide sequence downstream of the mutated region. The change in the reading frame results in a change in intrinsically encoded amino acid sequence, and this causes a conformational change and the like of the encoded protein and the mutated gene becomes a TYLCV resistance gene. In one aspect, this mutation is preferably a mutation of a nucleotide other than the 3rd nucleotide of a codon. There is no particular limitation on the number of replaced, deleted, added, and/or inserted nucleotides as long as the TYLCV resistance gene is obtained. For example, the number of nucleotides may be 1 to 5, 1 to 3, or 1 to 2.

The mutation of the TYLCV resistance gene is preferably at least one mutation selected from (a) to (d) above. The above-mentioned mutations (a) to (d) are not alternatives; for example, there are cases where the mutation (a) or (b) occurs as a result of the mutation (c) or (d).

Specific examples of mutation in RLK2 gene and gene homologous thereto include mutations (a) to (d) below:
(a) a frameshift mutation,
(b) a nonsense mutation,
(c) a loss of continuous or non-continuous 3n nucleotides (wherein n = 1 to 99), and
(d) a replacement, deletion, addition, and/or insertion of 1 or more nucleotides.

The frameshift mutation (a) and the nonsense mutation (b) are the same as described above.
(c) The loss of continuous or non-continuous 3n nucleotides (wherein n = 1 to 99, therefore, 3n nucleotides are 3 to 297 nucleotides) results in a change in the amino acid sequence encoded downstream of this lost region. Due to the occurrence of such a change, the mutated gene becomes a TYLCV resistance gene.
(d) The replacement, deletion, addition, and/or insertion of 1 or more nucleotides is the same as described above.

The above-mentioned mutations (a) to (d) are not alternatives; for example, there are cases where the mutation (a) or (b) occurs as a result of the mutation (c) or (d).

The mutation of the TYLCV resistance gene is preferably at least one mutation selected from (a) to (d) above, more preferably at least one mutation selected from (c) and (d) above, still more preferably an in-frame mutation, namely a mutation which does not cause a shift in the reading frame of a codon and does not cause a knockout of the gene, which is at least one mutation selected from (c) and (d') below.
(c) a loss of continuous or non-continuous 3n nucleotides (wherein n = 1 to 99), and
(d') an insertion of continuous or non-continuous 3n nucleotides (wherein n = 1 to 2).

In a preferred embodiment, there is no particular limitation with respect to the value of n in (c) a loss of continuous or non-continuous 3n nucleotides as long as n is within the range of 1 to 99. For example, n may be 4, 30, 45, 47, 59, etc. and the loss of 3n nucleotides is the loss of 6, 12, 90, 141, or 177 nucleotides. The lower limit value of n is 1, preferably 30, and the upper limit value of n is 99, preferably 59. The reason why the value of n within the range of 1 to 99 is preferred will be explained below.

In a preferred embodiment, (d') an insertion of continuous or non-continuous 3n nucleotides (wherein n = 1 or 2, for example, 3n nucleotides is 3 or 6 nucleotides) causes a change in the length or structure of an amino acid sequence encoded by a nucleotide sequence downstream of the insertion. Occurrence of such a change result in a TYLCV resistance gene.

There is no particular limitation with respect to the mutation in the genome of the solanaceous plants as long as the mutation inhibits the gene expression of the mutated gene or makes the protein encoded by the mutated gene to be non-functional for TYLCV, and imparts TYLCV resistance to the plants and also causes no considerable damage to the life or growth of the plants.

Next, the mutations are explained in detail.

In one aspect, when the solanaceous plants have a mutation in the DCL3 gene, the mutation is preferably in exon 8 (SEQ ID NO:2) of the DCL3 gene, and more preferably in a region comprising the 220th to 239th nucleotides in exon 8, that is, the region comprising CCGAGGTGGCTCTGATGTGC as set forth in SEQ ID NO:3. When the solanaceous plants have a mutation in the gene homologous to the DCL3 gene, the mutation is preferably in a region of the homologous gene which corresponds to the nucleotide sequence as set forth in SEQ ID NO:2, and more preferably in a part of the above region which corresponds to the nucleotide sequence as set forth in SEQ ID NO:3. Further, when a mutation is present in a region as set forth in SEQ ID NO:3 or a region corresponding thereto, other mutations may exist in other parts of exon 8.

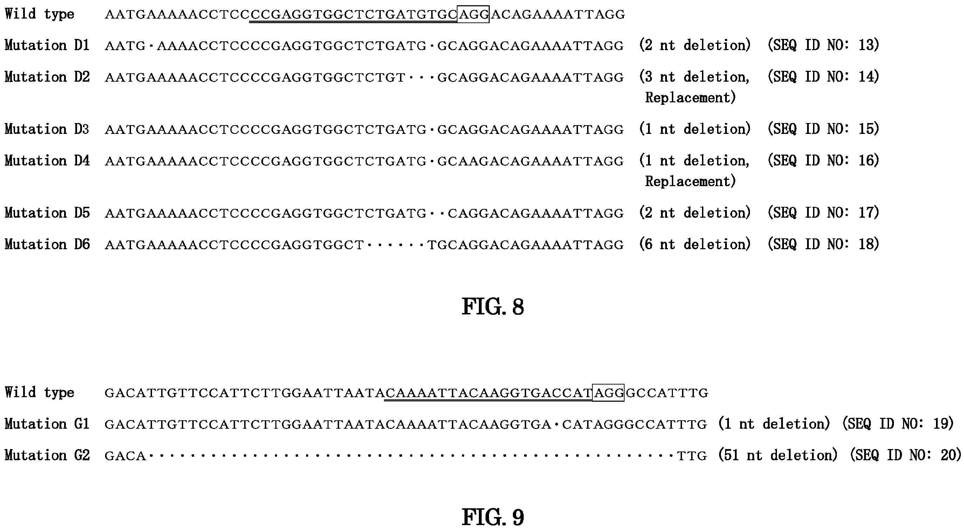

When the solanaceous plants have a mutation in exon 8 as set forth in SEQ ID NO:3 or a region corresponding to the sequence, the mutation is preferably a deletion of 1 nucleotide, deletion of 2 nucleotides, deletion of 3 nucleotides, deletion of 6 nucleotides or replacement of a nucleotide. Specific examples of such mutations in exon 8 are: deletion of 1 nucleotide which is the 210th nucleotide, deletion of 1 nucleotide which is the 237th nucleotide, deletion of 3 nucleotides which are the 235th to 237th nucleotides, deletion of 2 nucleotides which are the 237th to 238th nucleotides, deletion of 6 nucleotides which are the 231 st to 236th nucleotides, replacement of 234th nucleotide (e.g., replacement of A (adenine) with T(thymine)), and replacement of 241st nucleotide (e.g., replacement of G (guanine) with A). Specifically, the mutations are: deletion of 1 nucleotide at each of 210th and 237th positions of exon 8 (Mutation D1), replacement of 234th A with T and deletion of 3 nucleotides at 235th to 237th positions of exon 8 (Mutation D2), deletion of 1 nucleotide at 237th position of exon 8 (Mutation D3), deletion of 1 nucleotide at 237th position and replacement of 241st G with A of exon 8 (Mutation D4), deletion of 2 nucleotides at 237th to 238th positions of exon 8 (Mutation D5), and deletion of 6 nucleotides at 231st to 236th positions of exon 8 (Mutation D6) (see FIG. 8). Specific examples of mutated nucleotide sequences of exon 8 are shown in SEQ ID NOs: 13 to 18.

In one aspect, when the solanaceous plants have a mutation in the 4CL06 gene, the mutation is preferably in the 4CL06 gene (SEQ ID NO:4) on chromosome 6, more preferably in a region corresponding to exon 2 (SEQ ID NO:5) of the 4CL06 gene, and still more preferably in a region comprising the 286th to 305th nucleotides in exon 2, that is, the region comprising CAAAATTACAAGGTGACCAT as set forth in SEQ ID NO:6. When the solanaceous plants have a mutation in the gene homologous to the 4CL06 gene, the mutation is preferably in a region of the homologous gene which corresponds to the nucleotide sequence as set forth in SEQ ID NO:5, and more preferably in a part of the above region which corresponds to the nucleotide sequence as set forth in SEQ ID NO:6. Further, when a mutation is present in a region as set forth in SEQ ID NO:6 or a region corresponding thereto, other mutations may exist in other parts of exon 2.

When the solanaceous plants have a mutation in exon 2 as set forth in SEQ ID NO:5 or a region corresponding to the sequence, the mutation is preferably a deletion of 1 nucleotide or deletion of 51 nucleotides. Specific examples of such mutations in exon 2 are deletion of 1 nucleotide which is the 302nd nucleotide (Mutation G1), and deletion of 51 nucleotides which are the 263rd to 313rd nucleotides (Mutation G2) (see FIG. 9). Specific examples of mutated nucleotide sequences of exon 2 are shown in SEQ ID NOs: 19 and 20.

The present embodiment relates to the TYLCV resistance genes, which are: the mutated DCL3 gene *per se* having the mutated exon 8 as set forth in any one of SEQ ID NOs: 13 to 18, and the mutated 4CL06 gene *per se* having the mutated exon 2 as set forth in any one of SEQ ID NOs: 19 to 20. These genes are preferably the mutated DCL3 gene having a cDNA sequence which either comprises the nucleotide sequence as set forth in any one of SEQ ID NO:21 to 26 or consists of the nucleotide sequence as set forth in any one of SEQ ID NO:21 to 26, or the mutated 4CL06 gene having a cDNA sequence which either comprises the nucleotide sequence as set forth in SEQ ID NO:27 or 28 or consists of the nucleotide sequence as set forth in SEQ ID NO:27 or 28. The present embodiment also relates to the use of the above-mentioned mutated DCL3 gene and/or mutated 4CL06 gene, for imparting TYLCV resistance to the solanaceous plants.

In one aspect, when the solanaceous plants have a mutation in the RLK2 gene, the mutation is preferably in the RLK2 gene (SEQ ID NO:29) on chromosome 3, more preferably in a region a corresponding to exon 1 (SEQ ID NO:30) in the RLK2 gene, still more preferably in exon 1 (nucleotide sequence shown in SEQ ID NO:30) or a region corresponding thereto, and still more preferably in the nucleotide sequence as set forth in SEQ ID NO:36 in exon 1. When the solanaceous plants have a mutation in the gene homologous to the RLK2 gene, the mutation is preferably in a region of the homologous gene which corresponds to the nucleotide sequence as set forth in SEQ ID NO:29, more preferably in a part of the region which corresponds to the nucleotide sequence as set forth in SEQ ID NO:30, and still more preferably in a region corresponding to the nucleotide sequence as set forth in SEQ ID NO:36.

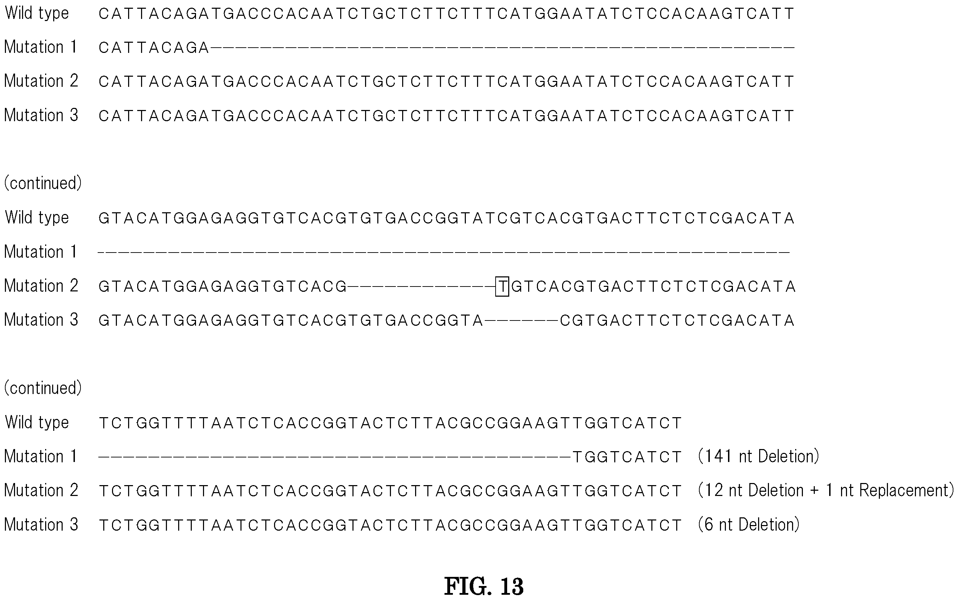

When the solanaceous plants have a mutation in exon 1 as set forth in SEQ ID NO:30 or a region corresponding to the sequence, the mutation is preferably a deletion of 3n (multiples of 3, in which n is an arbitrary number) nucleotides. Specific examples of such mutations in exon 1 are: deletion of 141 nucleotides (SEQ ID NO:36) (Mutation 1 below), deletion of 12 nucleotides (SEQ ID NO:37) (Mutation 2 below), and deletion of 6 nucleotides (SEQ ID NO:41) (Mutation 3 below) (see FIG. 13). Mutation 2 and Mutation 3 are deletions in the nucleotide sequence shown in SEQ ID NO:36, and each of Mutations 1 to 3 is an in-frame mutation. Further, Mutation 2 also includes a single nucleotide replacement of C to T at a position (281st nucleotide) flanking the 12-nucleotide deletion. Specific examples of mutated nucleotide sequences of exon 1 are shown in SEQ ID NOs:34, 35 and 40.

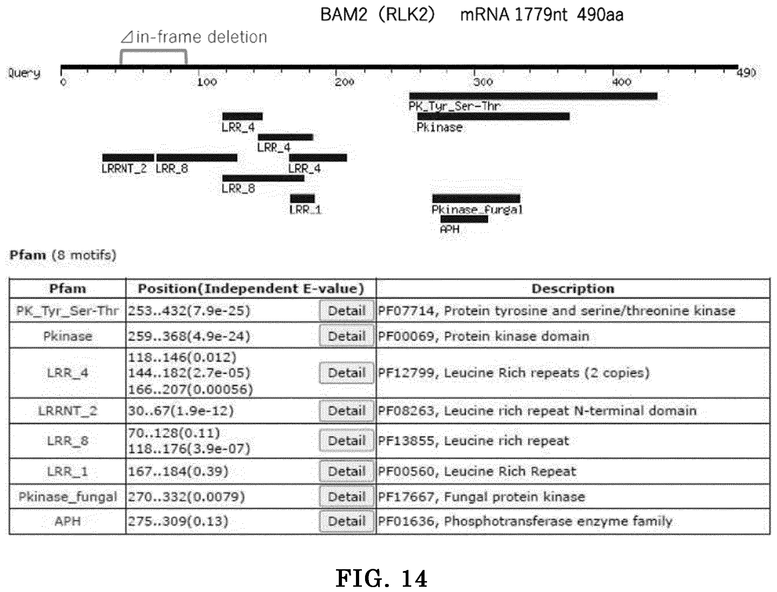

The mutations in the solanaceous plants are not limited to the specific examples mentioned above. For example, when the mutation in the RLK2 gene is a loss of continuous or non-continuous 3n nucleotides (i.e., (c) above), n is preferably a value selected from 1 to 99. Although not bound by any theory, the reason why n in the range of from 1 to 99 is preferred is presumed as follows. When the deletion is at least 3 nucleotides (i.e., n = 1), the mutation can be induced in-frame of the RLK2 gene. Further, as shown in FIG. 14, plurality of predicted motifs are present in the RLK2 amino acid sequence, and above Mutation 1 (loss of 141 nucleotides, i.e., n = 47) deletes a first half of the region from motif LRRNT_2 to motif LRR_8 without destroying the main structures, such as a kinase domain. Shortening the downstream portion of Mutation 1 to obtain a deletion of about 90 nucleotide (i.e., n = 30) is also considered effective for imparting virus resistance because the deleted region includes Mutation 2 and also reaches the motif LRR_8.

The present embodiment relates to the TYLCV resistance genes, which are: the mutated RLK2 gene *per se* having the mutated exon 1 as set forth in any one of SEQ ID NOs:34, 35 or 40. These genes are preferably the mutated RLK2 gene having a cDNA sequence which either comprises the nucleotide sequence as set forth in any one of SEQ ID NO:38, 39 or 42 or consists of the nucleotide sequence as set forth in any one of SEQ ID NO: 38, 39 or 42. The present embodiment also relates to the use of the above-mentioned mutated RLK2 gene for imparting TYLCV resistance to the solanaceous plants.

It should be noted that the mutations in the solanaceous plants are not limited to the above-mentioned regions, and mutations may exist in other regions of the DCL3 gene and/or the 4CL06 gene and/or the RLK2 gene, and other genes, as long as TYLCV resistance is maintained.

In one aspect, the mutation in the gene of the solanaceous plants is preferably a gene mutation introduced by genome editing techniques, such as the below-mentioned CRISPR system.

The mutated gene in the genome may be homozygous in which the mutation exists in both two alleles, or heterozygous in which the mutation exists in one of the alleles, but homozygous mutation is preferred. This is because properties imparted by the mutated gene are more strongly exhibited by a homozygous mutation in which two alleles are characterized by the same mutated sequence.

### (Virus resistant solanaceous plant and parts thereof)

The virus (e.g., TYLCV) resistant solanaceous plants of the present embodiment may be solanaceous plants with complex resistance showing resistance against viruses other than Begomoviruses and bacteria, as long as the plants show resistance against the viruses of genus *Begomovirus* causing tomato yellow leaf curl symptoms (e.g., TYLCV). Specific examples of other viruses include all potyviruses (PVY, etc.); viruses belonging to the genera *Bymovirus* and *Sobemovirus* and having a VPg similar to that of the PVY at their 5' terminus, in which a mutation in a translation initiation factor has been reported to impart resistance against these viruses; and viruses belonging to the genus *Carmovirus* in which a mutation in a translation initiation factor has been reported to impart resistance against these viruses.

In one aspect, the present embodiment relates to parts of the virus (TYLCV) resistant solanaceous plants. Such parts include not only parts collected from the solanaceous plants having the above-mentioned characteristics, and their progenies or clone plants, but also derivatives obtained from plant bodies or parts thereof. Specific examples of the parts include organs, such as fruits, shoots, roots, burgeons, and anthers; and plant tissues and cells. The parts may take any form, such as a suspension culture, protoplast, germ, callus tissue, lamina, gametophyte, sporophyte, pollen or microspore. An example of a derivative of the solanaceous plant is seeds.

In the present embodiment, the part of the virus resistant solanaceous plants may be a scion, rootstock, etc. used for grafting. Further, in one aspect, the present embodiment relates to plant cells (including calluses) which can regenerate the above-mentioned TYLCV resistant solanaceous plants, and the virus resistant solanaceous plants of the present embodiment also include such plants obtained from plant cells.

Parts of the solanaceous plants having virus resistance are preferably fruits which are edible fresh or useful for processing. In addition, the parts are preferably seed which are useful for progeny production and the like.

### (Processed material of solanaceous plants or parts thereof)

In one aspect, the present embodiment relates to a processed material of the solanaceous plants or the parts thereof. There is no particular limitation with respect to the processed materials, and examples include edible, industrial, and medical processed materials, and preferably the processed materials are edible materials.

For example, when the solanaceous plant having virus resistance is a tomato, examples of edible processed materials of tomato include canned tomatoes, tomato pastes, ketchups, tomato sauces, tomato soups, dried tomatoes, tomato juices, tomato powders, and tomato concentrates. A nutritional supplementary food (supplement) made from tomatoes is also an example of a processed material.

### [II] Solanaceous plant cells having virus resistance

In one aspect, the present embodiment relates to Solanaceous plant cells having virus resistance.

The solanaceous plant cells of the present embodiment has a mutation in at least one gene selected from the group consisting of silencing associated dicer gene (DCL3 gene) and a gene homologous thereto, 4-coumarate-CoA ligase gene on chromosome 6 (4CL06 gene) and a gene homologous thereto, and receptor-like kinase gene on chromosome 3 (RLK2 gene). Details of these genes and their mutation are already explained above in connection with the virus resistant solanaceous plants.

Virus resistance of the solanaceous plant cells may be confirmed by the above-mentioned methods. For example, absence or presence of virus resistance can be confirmed by infecting plant cells with a virus (TYLCV and the like) using a routine procedure, and determining the amount of accumulated virus in plant cells by conventional methods, such as ELISA, PCR, and the like.

The virus resistant solanaceous plant cells of the present embodiment may be either cells isolated from the above-mentioned virus resistant solanaceous plants and their progenies or clone plants, or plant cells with introduced gene mutation which are obtained by the below-mentioned method for producing a virus resistant solanaceous plant. Further, there is no particular limitation on the form of the virus resistant solanaceous plant cells, and the plant cells include a suspension culture and protoplast.

There is no particular limitation on the type of plant cells as long as the cells are solanaceous plant cells. The cells are preferably the cells of tomato, eggplant, tobacco, hot pepper, or potato, and more preferably the cells of tomato, eggplant or potato, and most preferably the cells of tomato.

In one aspect, the present embodiment relates to solanaceous plant bodies and parts thereof containing the above-mentioned solanaceous plant cells and having virus resistance. The solanaceous plant bodies and parts thereof include plant bodies or parts, such as tissues and organs, which have been regenerated from plant cells carrying an introduced gene mutation. Parts of a plant body regenerated from plant cells are also parts containing the above-mentioned solanaceous plant cells. The details of the parts are the same as those mentioned above in connection with the virus resistant solanaceous plants.

Further, parts of the solanaceous plants are preferably fruits which are edible fresh or useful for processing. In addition, the parts are preferably seeds which are useful for progeny production and the like.

In one aspect, the present embodiment relates to a processed material of the solanaceous plants or the parts thereof. There is no particular limitation with respect to the processed materials, and examples include edible, industrial, and medical processed materials, and preferably the processed materials are edible materials.

### [III] Method for producing a virus resistant solanaceous plant

In one aspect, the present embodiment relates to a method for producing a virus resistant solanaceous plant. Specifically, the method comprises the following steps:
selecting at least one gene from a group consisting of DCL3 gene, which is silencing associated dicer gene, and a gene homologous thereto; 4CL06 gene, which is 4-coumarate-CoA ligase gene on chromosome 6, and a gene homologous thereto; and RLK2 gene (Solyc03g043770), which is receptor-like kinase gene on chromosome 3, and a gene homologous thereto exclusive of RLK1 gene (Solyc02g091840);
introducing a mutation into the selected gene in a genome, in which the introduced mutation is either a mutation inhibiting an expression of the mutated gene or a mutation making a protein encoded by the mutated gene to be non-functional for a virus of genus *Begomovirus* causing tomato yellow leaf curl symptoms (e.g., TYLCV); and
selecting a solanaceous plant having resistance to the virus.

Firstly, a target gene for introducing a mutation is selected. At least one gene is selected from the group consisting of the DCL3 gene and a gene homologous thereto, the 4CL06 gene and a gene homologous thereto, and the RLK2 gene and a gene homologous thereto. The selected gene may be single gene or a combination of two or more different genes. Details of the genes are the same as those explained above in connection with the virus resistant solanaceous plants.

Next, the method for producing a virus resistant solanaceous plant is explained in detail taking TYLCV as an example.

Secondly, a mutation is introduced into the selected gene. Methods for introducing a mutation into a genomic gene can be broadly classified into two methods exemplified below.
(1) Direct genome editing: A method in which a plant carrying a TYLCV resistance gene is produced by directly editing a plant genome carrying the DCL3 gene, 4CL06 gene or RLK2 gene functional for TYLCV to introduce a mutation into a desired site at a pin point.
(2) Introduction of mutated gene: A method which combines the following steps (A) and (B): (A) TYLCV resistance gene is produced, and introduced into a plant by using an appropriate promotor; and (B) among endogenous genes of a plant which correspond to the TYLCV resistance gene produced in step (A) above, a gene functional for TYLCV is changed into a gene non-functional for TYLCV.

Each method is explained below.

### (1) Direct genome editing

Direct genome editing can be performed using conventional genome editing techniques which use a site-specific nuclease, such as CRISPR, TALEN or the like. When a double strand cleavage is introduced using a restriction enzyme capable of cleaving a specific site in a genome, various mutations are introduced at the time of repair due to a repair error. As a result, a mutation is introduced into a target gene (in the present embodiment, a gene encoding DCL3, 4CL06 or RLK2).

Preferably a CRISPR system, and more preferably a CRISPR/Cas9 system is used since these systems are capable of introducing mutation at high specificity and high efficiency. In the CRISPR/Cas9 system, a guide RNA (sgRNA) which has a sequence of about 20 nucleotides long and which is homologous to the target gene recognizes the target and Cas9 protein cleaves the double strand. During the repair of the resultant cleavage by non-homologous end-joining (NHEJ) repair cycle, a mutation is introduced into the target site due to a repair error.

Delivery of the Cas protein and sgRNA to a plant may be performed via a vector encoding the same by using methods well-known to those skilled in the art, such as an agrobacterium method, standard transfection, electroporation, particle bombardment and the like.

Briefly, as explained in Examples hereinbelow, the Cas protein and sgRNA are delivered to plants by constructing a binary vector with incorporated Cas gene and sgRNA, and transforming agrobacterium with the constructed vector, followed by transformation of plants with the agrobacterium (see, for example, Friedrich Fuser et al., "The Plant Journal," 2014, 79: 348-359; and Ryo Oosawa and Hiroshi Ezura, "Atarashii Shokubutu Ikushu Gijyutu wo Rikaisiyou - NBT (New plant breeding techniques) (Understanding New Plant Breeding Techniques - NBT (New plant breeding techniques))", International Academic Publishing Co., Ltd., 2013).

There is no particular limitation on the form of the plants being transformed with the agrobacterium, as long as the plant is capable of reproducing a plant body. Examples of such plant forms include a suspension culture, protoplast, leaf section, callus and the like. After removing the agrobacterium, the transfected plants can be cultured in a medium containing a antibiotic selected in accordance with the vector used, and a selection culture of a plant (section) having an incorporated desired gene may be performed based on antibiotic resistance.

The guide RNA may be designed to enable a highly efficient introduction of a mutation into a target site. In general, a site 3 nucleotides upstream of a 3-nucleotide sequence called a PAM sequence (which is NGG for the most popular Cas9 derived from *S*. *pyogenes*) is generally cleaved in a CRISPR system. Since the PAM sequence must exist immediately after the target sequence, the guide RNA may be designed so that the target sequence is located upstream of the PAM sequence.

When designing the guide RNA, GC content is preferably taken into consideration because higher the GC content, the higher is the cleavage efficiency. Further, the system may be designed to minimize non-specific cleavage by an off-target effect. In one aspect, when the plant is tomato, the guide RNA is preferably designed to have a nucleotide sequence which targets a specific sequence (e.g., SEQ ID NO:3) in exon 2 of chromosome 6 and/or a specific sequence (e.g., SEQ ID NO:6) in exon 8 of chromosome 8, and/or a specific sequence (e.g., SEQ ID NO:3) in exon 1 of chromosome 3.

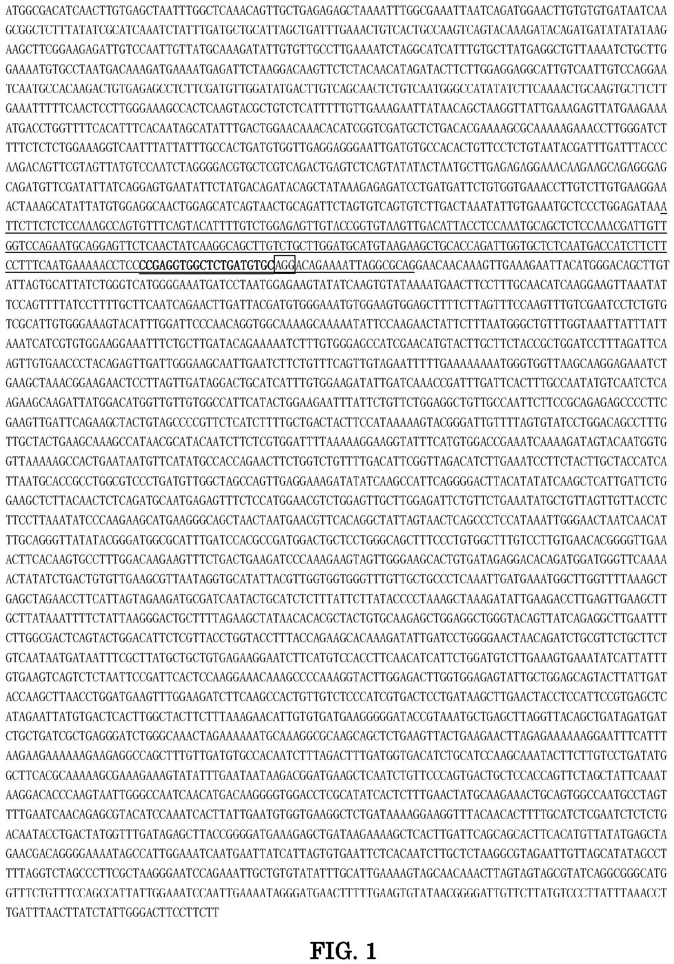

For example, in FIG. 1 which illustrates the cDNA sequence (SEQ ID NO: 1) of the DCL3 gene of a tomato, the guide RNA may be designed so that the PAM sequence is the boxed portion in exon 8 (i.e., single underlined portion of FIG. 1; SEQ ID NO:2) and the target is generally 20 nucleotides (i.e., double underlined portion of FIG. 1; SEQ ID NO:3) located upstream of the boxed 3 nucleotides. The direct genome editing of other solanaceous plants can be performed in the same manner as in tomatoes, by selecting a target from a region in a gene homologous to the DCL3 gene which corresponds to the nucleotide sequence as set forth in SEQ ID NO:3 and, then, selecting the PAM sequence and designing the guide RNA. Plants having TYLCV resistant DCL3 gene can be produced by introducing a mutation to the target site into the above-mentioned manner.

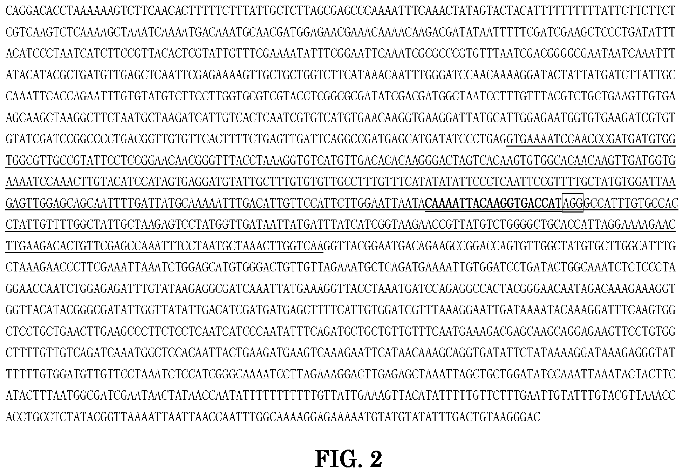

In FIG. 2 which illustrates the cDNA sequence (SEQ ID NO:4) of the 4CL06 gene present on chromosome 6 of a tomato, the guide RNA may be designed so that the PAM sequence is the boxed portion in exon 2 (i.e., single underlined portion of FIG. 2; SEQ ID NO:5) and the target is generally 20 nucleotides (i.e., double underlined portion of FIG. 2; SEQ ID NO:6) located upstream of the boxed 3 nucleotides. The direct genome editing of other solanaceous plants can be performed in the same manner as in tomatoes, by selecting a target from a region in a gene homologous to the 4CL06 gene which corresponds to the nucleotide sequence as set forth in SEQ ID NO:6 and, then, selecting the PAM sequence and designing the guide RNA. Plants having TYLCV resistant 4CL06 gene can be produced by introducing a mutation into the target site in the above-mentioned manner.

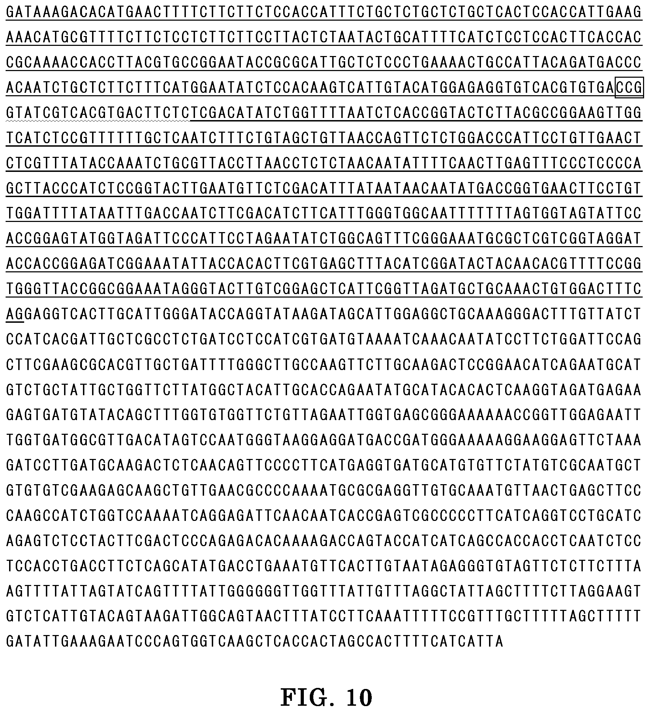

In FIG. 10 which illustrates the cDNA sequence (SEQ ID NO:29) of the RLK2 gene present on chromosome 3 of a tomato, the guide RNA may be designed so that the PAM sequence is the boxed portion in exon 1 (i.e., single underlined portion of FIG. 10; SEQ ID NO:30) and the target is generally 20 nucleotides (i.e., wavy underlined portion of FIG. 10; SEQ ID NO:31) located upstream of the boxed 3 nucleotides. The direct genome editing of other solanaceous plants can be performed in the same manner as in tomatoes, by selecting a target from a region in a gene homologous to the RLK2 gene which corresponds to the nucleotide sequence as set forth in SEQ ID NO:31 and, then, selecting the PAM sequence and designing the guide RNA. Plants having TYLCV resistant RLK2 gene can be produced by introducing a mutation into the target site in the above-mentioned manner.

When a double strand cleavage is introduced into a single site by the CRISPR system, about 20 nucleotides are repaired and a mutation is considered to be introduced by the repair error. Therefore, in one aspect, the mutation in the TYLCV resistance gene of the present embodiment is a mutation of continuous or non-continuous 3n nucleotides (wherein n = 1 to 20, preferably n = 1 to 10, more preferably n = 1 to 3). The introduced mutation is not necessarily limited to the target site. In other words, mutation may be present in regions other than the target site.

Further, the present embodiment relates to the guide RNAs and vectors comprising the guide RNA which are used for producing the TYLCV resistant solanaceous plant. The sequences of the guide RNAs are as mentioned above. The present embodiment also relates to kits comprising the guide RNA. The kits may comprise a site-specific nuclease and the like which are necessary for genome editing using the CRISPR system, and the kit may be used for producing the TYLCV resistant solanaceous plants.

### (2) Introduction of mutated gene

Introduction of mutated gene is a method which combines the following steps (A) and (B).
(A) TYLCV resistance gene is produced, and introduced into a plant by using an appropriate promotor; and
(B) among endogenous genes of a plant which correspond to the TYLCV resistance gene produced in step (A) above, a gene functional for TYLCV is changed into a gene non-functional for TYLCV.

There is no particular limitation on the order for performing the above steps (A) and (B) as long as the steps cause no fatal damage to the plants, and step (B) may be performed before step (A). It should be noted that a method which performs step (B) alone at a specific part of the plant is the method (1) Direct Genome Editing.

In step (A), a mutated gene encoding DCL3 protein non-functional for TYLCV, and/or a mutated gene encoding 4CL06 protein non-functional for TYLCV is/are produced, and introduced into plants using an appropriate promotor. Production of the mutated gene may be performed by conventional methods well-known in the art. For example, a nucleotide sequence having a desired mutation may be synthesized and amplified by PCR, etc. The mutations introduced herein are the same as those explained above in connection with the TYLCV resistant solanaceous plants.

Introduction of the produced mutated gene into plants may be also performed by conventional methods well-known in the art. In brief, the introduction may be performed using a vector containing the mutated gene, for example by a polyethylene glycol method, electroporation, agrobacterium method, particle gun method and the like. The mutated gene introduced herein is the TYLCV resistance gene obtained by introducing a mutation into the DCL3 gene (or the gene homologous thereto) and/or 4CL06 gene (or the gene homologous thereto) derived from a solanaceous plant, and the TYLCV resistance gene can be derived from solanaceous plants of different species.

There is no particular limitation on the form of the plants used for introducing the above-mentioned vector, as long as the plant is capable of reproducing a plant body. Examples of such plant forms include a suspension culture, protoplast, leaf section, callus and the like.

Next, in step (B), among the endogenous DCL3 gene (or the gene homologous thereto) and/or 4CL06 gene (or the gene homologous thereto) of the plant, a gene which is functional for TYLCV is changed into a gene which is non-functional for TYLCV. Step (B) may be performed using conventional methods which are used for introducing mutation into plants. Examples of such methods include treatments with a mutagen, such as ion beam or EMS (Ethyl methanesulfonate). Step (B) may be performed using the above-mentioned genome editing techniques, such as CRISPR and TALEN. Desirably, all of the genes functional for TYLCV among the endogenous DCL3 and/or 4CL06 are changed into a state which is non-functional for TYLCV.

Subsequently, a plant body is reproduced from the parts (such as, leaf sections or plant cells) of the plant carrying the TYLCV resistance gene. Reproduction of the plant body may be performed by well-known conventional methods in accordance with the type of the plant. For example, reproduction may be performed by making reference to Sun H.J. et al., "Plant Cell Physiol.," 2006, 47: 426 and the like for tomatoes, and Jefferson R.A. et al., "EMBO J.," 1987, 6: 3901 and the like for tobaccos.

Further, solanaceous plants having resistance against TYLCV are selected from the reproduced plants. Such a selection may be performed by the above-mentioned methods for confirming TYLCV resistance. For example, plants having TYLCV resistance may be selected by infecting the plants with TYLCV using a routine procedure, and detecting the accumulation of TYLCV in the plants by conventional methods, such as ELISA, PCR and the like. Alternatively, solanaceous plants having TYLCV resistance may be selected by determining the presence or absence of TYLCV infection symptoms (yellowing, curing (curving), dwarf, chlorosis, etc. of leaves) in the TYLCV infected plants.

The solanaceous plants produced by the above-mentioned methods include tomato, eggplant, tobacco, hot pepper, potato and the like, preferably tomato, eggplant, and potato, and more preferably tomato.

In one aspect, the present embodiment relates to solanaceous plants produced by the above-mentioned method. Such solanaceous plants are the same as the TYLCV resistant solanaceous plants explained above.

Once the TYLCV resistant solanaceous plants carrying the TYLCV resistance gene are obtained, progenies and clones of such plants may be obtained by conventional methods. Therefore, the TYLCV resistant solanaceous plants of the present embodiment include such progenies and clones.

In one aspect, the present embodiment relates to a method for producing a bred progeny of the TYLCV resistant solanaceous plant, comprising: self-pollination or cross-pollination of TYLCV resistant solanaceous plant (first generation) or its progeny. The self-pollination or cross-pollination of a plant can be performed by any conventional methods well-known in agricultural breeding sector, and can be performed under either natural or artificial conditions. The progeny obtained in this manner may be subjected to self-pollination or cross-pollination to produce a further progeny.

A solanaceous plant used to crossbreed with the first generation or later progeny by cross-pollination may be a solanaceous plant having either the same mutation in the same gene, a different mutation in the same gene, or a mutation in a different gene. For example, it is possible to crossbreed 2 different plants selected from a plant having mutation in the DCL3 gene and a plant having mutation in the 4CL06 gene. It is also possible to perform breeding several times to produce a plant carrying mutation in 3 or more genes.

### Examples

### [Example 1]

### Production of recombinant agrobacterium A for introducing mutation into DCL3 gene

Guide RNA recognition site was designed in exon 8 (SEQ ID NO:2) of DCL3 gene (Solyc08g067210) which is said to be present on chromosome 8 of tomatoes. Double stranded DNA corresponding to the designed site of 20 nucleotide-long (SEQ ID NO:3: CCGAGGTGGCTCTGATGTGC) was synthesized and inserted into restriction enzyme BbsI site of vector pUC19_AtU6oligo (obtained from National Research and Development Agency, National Institute of Agrobiological Sciences), thereby constructing recombinant vector A. cDNA sequence of the DCL3 gene present on chromosome 8 of wild type tomatoes is shown in FIG. 1 and SEQ ID NO: 1.

A cassette site containing the guide RNA region was cutout from the constructed recombinant vector A and inserted into restriction enzyme I-*Sce*I site of binary vector pZD_OsU3gYSA_HolgerCas9_NPTII, thereby obtaining recombinant binary vector A. Agrobacterium LBA4404 (manufactured and sold by Takara Bio Inc) was transformed with the binary vector A by a conventional method to obtain recombinant agrobacterium A.

### [Example 2]

### Production of recombinant agrobacterium B for introducing mutation into 4CL06 gene

Guide RNA recognition site was designed in exon 2 (SEQ ID NO:5) of 4CL06 gene (Solyc06g068650) which is said to be present on chromosome 6 of tomatoes. Double stranded DNA corresponding to the designed site of 20 nucleotide-long (SEQ ID NO:6: CAAATTACAAGGTGACCAT) was synthesized and inserted into restriction enzyme BbsI site of vector pUC19_AtU6oligo (obtained from National Research and Development Agency, National Institute of Agrobiological Sciences), thereby constructing recombinant vector B. cDNA sequence of the 4CL06 gene present on chromosome 6 of wild type tomatoes is shown in FIG. 2 and SEQ ID NO:4.

A cassette site containing the guide RNA region was cutout from the constructed recombinant vector B and inserted into restriction enzyme I-*Sce*I site of binary vector pZD_OsU3gYSA_HolgerCas9_NPTII, thereby obtaining recombinant binary vector B. Agrobacterium LBA4404 (manufactured and sold by Takara Bio Inc) was transformed with the binary vector B by a standard method to obtain recombinant agrobacterium B.

### [Example 3]

### Transformation of tomatoes

A conventional variety Moneymaker (hereinafter abbreviated to "MM") and a personal variety S were used for transformation of tomatoes. Transformation of tomatoes using an agrobacterium was performed in accordance with a common textbook (for example, Yataka Tabei ed., "Keishitutenkan Purotokoru <Shokubutu-hen> (Protocols for plant transformation)", Kagaku-Dojin Publishing Company, INC., 2012). Specifically, either sections of cotyledons obtained by germination of tomato seeds in sterile medium, or sterilized sections of cotyledons or leaves obtained by usual seeding were prepared. Next, each of the recombinant agrobacteria A and B obtained in Examples 1 and 2, respectively, was cultured individually until the turbidity of the culture liquid reached 0.1 to 1.0, and the sections were immersed in the culture liquid for about 10 minutes, to thereby infect the sections with the agrobacteria.

On 3 days post infection, the agrobacteria were removed. Tomato leaf sections were transferred to Murashige and Skoog medium (may be abbreviated to MS medium) (a medium obtained by adding 3% sucrose, 1.5 mg/L zeatin and 1% agar to MS basic medium) supplemented with carbenicillin (100 to 500 mg/ml) and kanamycin (20 to 100 mg/ml). The leaf sections were subjected to selection culture at 25 °C under light (16 hours light/8 hours dark). Leaf sections were passaged by changing the medium every 10 days to 2 weeks from the start of the culture, thereby promoting the formation of calluses from the leaf sections. The passage was continued further to induce the formation of adventitious buds.

When the size of the adventitious buds reached several centimeters, the buds were transferred to a rooting medium (a medium obtained by adding 1.5% sucrose, 1% agar, 50 to 250 mg/ml carbenicillin, 20 to 100 mg/ml kanamycin, and optionally naphthalene acetic acid (NAA), to MS basic medium) and cultured for 1 to 3 months while passaging every month.

All cultures until the culture in the rooting medium, were performed in sterile condition. The rooted plants were taken out from the sterile rooting medium and transferred to a conventional pot soil obtained by mixing black soil, Akadama soil and the like, and cultivated in the pot. The thus obtained reproduced plants were first generation transgenic plants (hereinbelow, sometimes abbreviated to "T0").

### [Example 4]

### Selection of Gene Edited Subculture

For confirming the presence or absence of gene recombination and edited site (site with deletion, insertion or replacement of a nucleotide) in the target gene of the first generation transgenic plants, the desired sites were amplified by PCR using the following primers: primer 1 (CTCAACTATCAAGGCAGCTTG (SEQ ID NO:7)) and primer 2 (GATAAAAGCTCCTCCTTGCCGAGT (SEQ ID NO:8)) for the region in the DCL3 gene (Solyc08g067210); and primer 3 (GGTTTACCTAAAGGTGTCATGT (SEQ ID NO:9)) and primer 4 (CAGTGTCTTCAAGTTCTTTTCC (SEQ ID NO:10)) for the region in the 4CL06 gene (Solycc06g068650).

PCR was performed using "KOD Plus Neo" manufactured and sold by TOYOBO CO., LTD., and DNA was amplified in accordance with the enclosed manual.

Next, the amplified fragments were treated with a restriction enzyme having its cleavage site in the target site of the fragments to confirm whether the amplified fragments are cleaved by the restriction enzyme or not. Specifically, *BstE*II was used for the 4CL06 gene. Amplified fragments will not be cleaved by the restriction enzyme when the restriction site is changed by recombination and editing of the gene. Occurrence of gene recombination and editing in the target gene was determined based on the non-cleavage of the amplified fragments (data not shown). Further, since there was no appropriate restriction enzyme sites within the target site of the DCL3 gene, the presence or absence of editing was confirmed by sequencing the nucleotide sequence of the site as in the same manner as in [Example 7] below.

As a result, in some of the reproduced plants, editing of a nucleotide sequence in the DCL3 gene or 4CL06 gene was confirmed and edited lines were selected. The edited lines of the DCL3 gene and the 4CL06 gene were named "DCL3" and "G27", respectively.

### [Example 5]

### Test 1 for confirming TYLCV resistance of DCL3 mutated tomato

In nature, TYLCV is transmitted by an insect vector which is whitefly and, therefore, plants are not infected by mechanical inoculation using sap from infected leaves. Viral infectious clone inoculation method using an agrobacterium was employed for efficiently infecting a plurality of samples with TYLCV. In this method, similarly to the method for gene transgenic, an agrobacterium binary vector harboring the whole TYLCV sequence incorporated therein (infectious clone) is produced, and an agrobacterium is transformed with the produced clone. The resultant recombinant agrobacterium is cultured in a liquid medium and then, the medium is replaced with 0.01M MES buffer (pH 5.7). The agrobacterium is inoculated by injection, infiltration or the like to plant bodies (i.e., tomato in this experiment) at their growing points, relatively young stems, or petioles, hence the cells in the vicinity of the infiltrated parts become infected with agrobacterium. When the infected agrobacterium introgress TYLCV DNA into the plant genome, the TYLCV DNA in the plant genome is expressed by the NOS promoter and the like designed in the binary vector. As a result, TYLCV particles are produced and viral infection is established. Such infectious clone inoculation imitated infection with mechanical inoculation because TYLCV genome also replicates inside the nucleus of a plant cell in nature.

Plants (T0) of DCL3 gene-edited line selected in Example 4 were grown in an isolated green house and were self-pollinated for collecting seeds. The seeds which are transgenic progeny (T1 or T2) are sown and grown. The thus obtained seedlings were inoculated with infectious clone of TYLCV-Israel strain by the above-mentioned test for confirming TYLCV resistance. A wild type variety MM free of introduced mutation was also tested as a control.

In the first infective clone inoculation test, observation of symptoms were performed on 21 days post inoculation. In the observation of symptoms, plants were defined as positive when typical symptoms of TYLCV infection, such as yellowing, curling (curving), dwarf, and/or chlorosis of the leaves, were observed. Regarding the edited lines of the DCL3 gene, on 21 days post inoculation, 6 plants out of 7 plants inoculated with the virus had no symptoms. On the other hand, regarding the control, 5 plants out of 6 plants inoculated with the virus showed symptoms (see Table 1 below).

Next, DNA was extracted from leaves of seedlings participating in the test and subjected to PCR analysis using TYLCV detection primer 5 (CCCTCTGGAATGAAGGAACA, SEQ ID NO: 11) and primer 6 (TTGAAAAATTGGRCTCTCAA, SEQ ID NO: 12). When the PCR products were confirmed by agarose gel electrophoresis using 1% agarose gel, virus was not detected in 3 out of 7 plants of the DCL3 edited line, but in the control, virus was detected in 5 out of 6 plants (see Table 1 below).

In the DCL3 edited lines, morbidity determined by observation of symptoms was lower than infection rate determined by PCR analysis. Accordingly, the mutation may have an effect of delaying the development of symptoms.

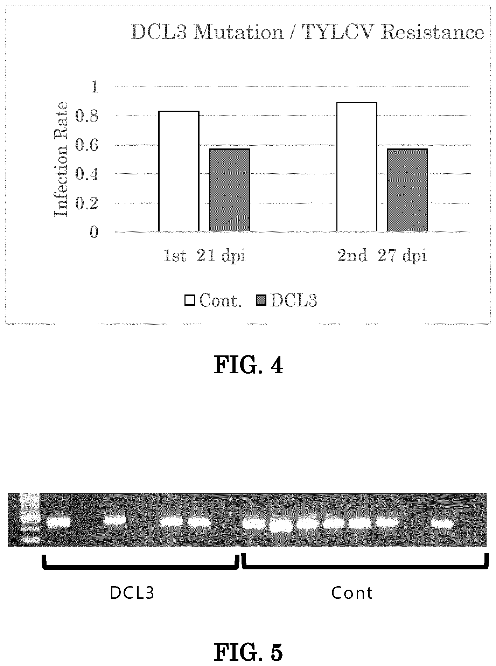

In addition, second TYLCV inoculation test was performed in the same manner as the first test using the T1 seedlings of the G27 line. As a result, during the observation of symptoms on 27 days post inoculation, 3 plants out of 7 plants inoculated with the virus had no symptoms. On the other hand, regarding the control, 8 plants out of 9 plants inoculated with the virus showed symptoms. As in the first test, DNA was extracted from leaves of seedlings participating in the test and subjected to PCR analysis using TYLCV detection primers. When the PCR products were confirmed by agarose gel electrophoresis, as in the observation of symptoms, virus was detected in only 4 out of 7 plants of the DCL3 edited line, and in 7 out of 8 control plants (see Table 1 and FIGS. 4 and 5).

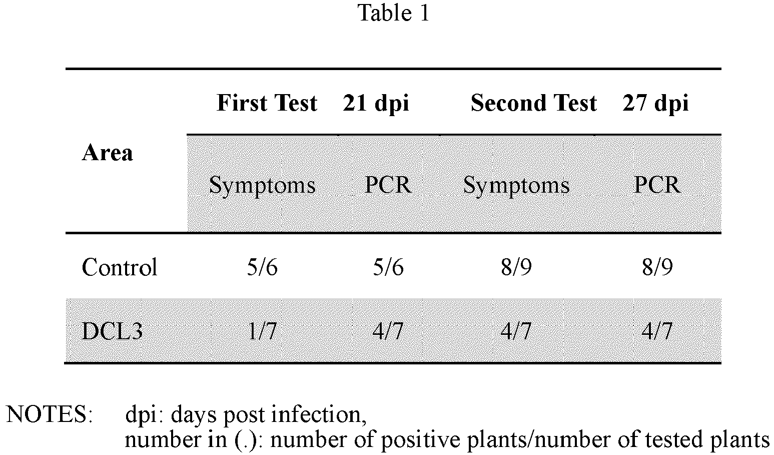

In the DCL3 edited line, results confirming the virus resistance was also obtained for T1 generation (data not shown) which is a generation prior to the T2 generation which has been confirmed to have the virus resistance, and inheritance of the TYLCV resistance by the mutation introduced into the DCL3 gene has been confirmed by such results. The test method used herein is a method with much higher infection pressure than a virus infection in nature by an insect vector and, therefore, the morbidity obtained in the present test is very high. In nature, the morbidity of a wild-type tomato plant (common tomato plant), such as that use as the control, is less than 30 %. Accordingly, the morbidity of the edited line in nature will be even lower and is suspected to be a level which causes no occurrence of the disease.

### [Example 6]

### Test for confirming TYLCV resistance of 4CL06 mutated tomato

As in Example 5, plants (T0) of 4CL06 gene-edited line G27 selected in Example 4 were grown in an isolated green house and were self-pollinated for collecting seeds. The seeds which are transgenic progeny (T1) are sown and grown. The thus obtained T1 seedlings were inoculated with infectious clone of TYLCV-Israel strain in the same manner as in Example 5.

In the first inoculation test, regarding the 4CL06 gene-edited line G27, 3 plants among 3 plants being inoculated with the virus showed no symptoms on 21 days post inoculation. On the other hand, regarding the control, all 6 virus inoculated plants showed symptoms. Further, DNA was extracted from leaves of seedlings participating in the test and subjected to PCR analysis using the above-mentioned TYLCV detection primers. When the PCR products were confirmed by agarose gel electrophoresis, virus was detected in only 1 out of 3 plants of the G27 edited line, and on the other hand, in all of the control plants (see Table 2 below). From these results, effect of delaying the development of symptoms has been confirmed for the mutation in the G27 line.

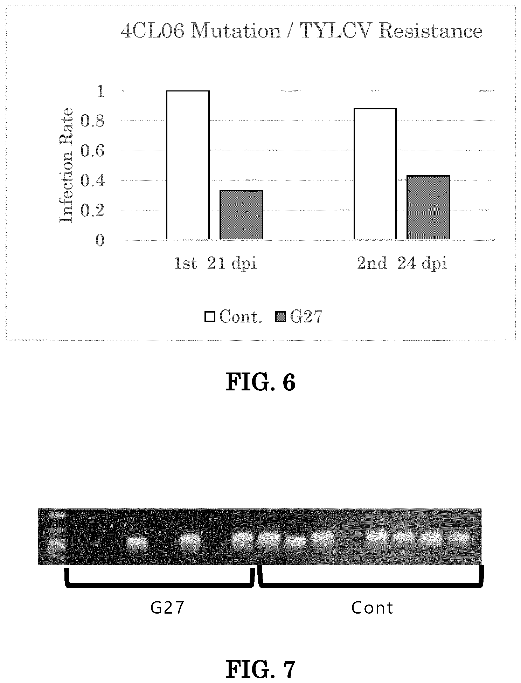

In addition, second TYLCV inoculation test was performed in the same manner as the first test using the T1 seedlings of the G27 line. As a result, during the observation of symptoms on 24 days post inoculation, with respect to the G27 line, 2 out of 7 plants had no symptoms. On the other hand, regarding the control, 7 out of 8 plants showed symptoms. As in the first test, DNA was extracted from leaves of seedlings participating in the test and subjected to PCR analysis using TYLCV detection primers. When the PCR products were confirmed by agarose gel electrophoresis, virus was detected in only 3 out of 7 plants of the G27 line, and in 7 out of 8 control plants (see Table 2 and FIGS. 6 and 7).

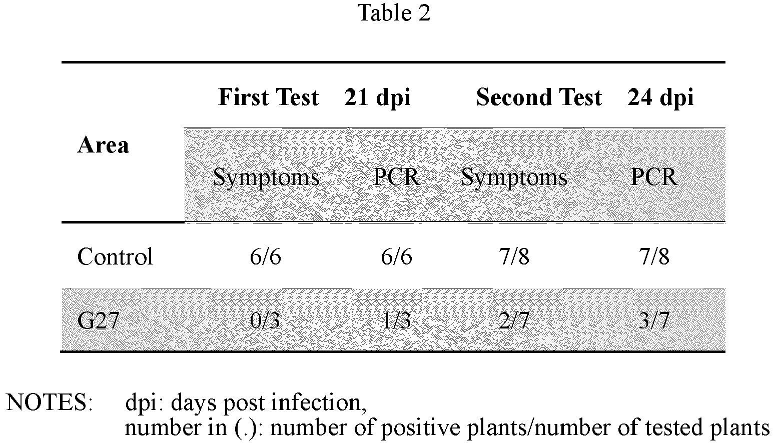

### [Example 7]

### Sequencing of TYLCV resistance DCL3 gene

A region around the DCL3 edited site (i.e., a region starting from the nearby 120th nucleotide to the 3' region of exon 8 (SEQ ID NO:2) of the DCL3 gene on tomato chromosome 8) of a TYLCV resistance T2 plant was amplified by PCR (T100 thermocycler, manufactured and sold by Bio-Rad laboratories Inc.) using the above-mentioned primer 1 and primer 2. The amplified fragments were cloned for determining the nucleotide sequence.

As a result, deletion or replacement of several nucleotides in the same region were confirmed. Sequences of the mutated regions found in exon 8 of the DCL3 gene are shown in FIG. 8, together with the corresponding region of the wild type nucleotide sequence as set forth in SEQ ID NO:3. Specifically, the following 6 mutations were detected. Mutation D1 is deletion of 1 nucleotide at each of 210th and 237th positions of exon 8, Mutation D2 is replacement of 234th A (adenine) with T (thymine) and deletion of 3 nucleotides at 235th to 237th positions of exon 8, Mutation D3 is deletion of 1 nucleotide at 237th position of exon 8, Mutation D4 is deletion of 1 nucleotide at 237th position and replacement of 241st G (guanine) with A (adenine) of exon 8, Mutation D5 is deletion of 2 nucleotides at 237th to 238th positions of exon 8, and Mutation D6 is deletion of 6 nucleotides at 231st to 236th positions of exon 8.

### [Example 8]

### Sequencing of TYLCV resistance 4CL06 gene

A region around the 4CL06 edited site (i.e., a region starting from the nearby 55th nucleotide to the 3' region of exon 2 (SEQ ID NO:5) of the 4CL06 gene on tomato chromosome 6) of a TYLCV resistance T1 plant was amplified by PCR (T100 thermocycler, manufactured and sold by Bio-Rad laboratories Inc.) using the above-mentioned primer 3 and primer 4. The amplified fragments were cloned for determining the nucleotide sequence.

As a result, deletion of several nucleotides in the same region were confirmed. Sequences of the mutated regions found in the 4CL06 gene are shown in FIG. 9, together with the corresponding region of the wild type nucleotide sequence as set forth in SEQ ID NO:6. Specifically, the following 2 mutations were detected. Mutation G1 is deletion of 1 nucleotide which is the 302nd nucleotide of exon 2, and Mutation G2 is deletion of 51 nucleotides which are the 263rd to 313rd nucleotides of exon 2.

### [Example 9]

### Production of tomato transformant having TYLCV resistance DCL3 gene and TYLCV resistance 4CL06 gene

A first generation transgenic plant is obtained by transfecting a tomato with an agrobacterium in the same manner as in Example 3. The agrobacterium used may be either a combination of the recombinant agrobacterium A obtained in Example 1 and the recombinant agrobacterium B obtained in Example 2; or agrobacterium F which is a recombinant obtained using binary vector F obtained by linking the guide RNA recognition sites of Examples 1 and 2.

Next, presence or absence of gene recombination and editing (deletion, insertion or replacement of a nucleotide) in the target genes, that is both the DCL3 gene and 4CL06 gene, of the first generation transgenic plant are confirmed in the same manner as described in Example 4. Edited line having mutations in both genes is selected.

The selected edited line is subjected to the test for confirming TYLCV resistance in the same manner as described in Examples 5 and 6. Production of tomato plant having the gene recombination and editing (deletion, insertion or replacement of a nucleotide) in both the DCL3 gene and 4CL06 gene, and acquiring TYLCV resistance may be confirmed by this test.

### [Example 10]

### Production of recombinant agrobacterium for introducing mutation into RLK2 gene

Guide RNA recognition site was designed in exon 1 (SEQ ID NO:30) of RLK2 gene (Solyc03g043770) which is said to be present on chromosome 3 of tomatoes. Here, target was a minus strand of the RLK2 gene. Double stranded DNA corresponding to the designed 20 nucleotide-long site (SEQ ID NO:31: GTATCGTCACGTGACTTCTC) was synthesized and inserted into restriction enzyme *Bbs*I site of vector pUC19_AtU6oligo (obtained from National Research and Development Agency, National Institute of Agrobiological Sciences), thereby constructing a recombinant vector. cDNA sequence of the RLK2 gene present on chromosome 3 of wild type tomatoes is shown in FIG. 10 and SEQ ID NO:29.

A cassette site containing the guide RNA region was cutout from the constructed recombinant vector and inserted into restriction enzyme I-*Sce*I site of binary vector pZD_OsU3gYSA_HolgerCas9_NPTII, thereby obtaining recombinant binary vector. Agrobacterium LBA4404 (manufactured and sold by Takara Bio Inc) was transformed with the binary vector B by a standard method to obtain recombinant agrobacterium.

### [Example 11]

### Transformation of tomatoes

A common pure bred variety Moneymaker (hereinafter abbreviated to "MM") and a personal heirloom variety S were used for transformation of tomatoes. Transformation of tomatoes using an agrobacterium was performed in accordance with a common textbook (for example, Yataka Tabei ed., "Keishitutenkan Purotokoru <Shokubutu-hen> (Protocols for plant transformation)", Kagaku-Dojin Publishing Company, INC., 2012). Specifically, either sections of cotyledons obtained by germination of tomato seeds in sterile medium, or disinfected sections of cotyledons or leaves obtained by usual seeding were prepared. Next, the recombinant agrobacterium obtained in Example 10 was cultured until the turbidity of the culture liquid reached OD₆₀₀ 0.1 to 1.0, and the sections were immersed in the culture liquid for about 10 minutes, to thereby infect the sections with the agrobacterium.

On 3 days post infection, the agrobacteria were removed. Tomato leaf sections were transferred to Murashige and Skoog medium (may be abbreviated to MS medium) (a medium obtained by adding 3% sucrose, 1.5 mg/L zeatin and 1% agar to MS basic medium) supplemented with carbenicillin (100 to 500 mg/ml) and kanamycin (20 to 100 mg/ml). The leaf sections were subjected to selection culture at 25 °C under light (16 hours light/8 hours dark). Leaf sections were passaged by changing the medium every 10 days to 2 weeks from the start of the culture, thereby promoting the formation of callus from the leaf sections. The passage was continued further to induce the formation of adventitious buds.

When the size of the adventitious buds reached several centimeters (like shoot), the buds were transferred to a rooting medium (a medium obtained by adding 1.5% sucrose, 1% agar, 50 to 250 mg/ml carbenicillin, 20 to 100 mg/ml kanamycin, and optionally naphthalene acetic acid (NAA), to MS basic medium) and cultured for 1 to 3 months while passaging every month.

All cultures until the culture in the rooting medium, were performed in sterile condition. The rooted plants were taken out from the sterile medium and transferred to a conventional pot soil obtained by mixing black soil, Akadama soil and the like, and cultivated in the pot. The thus obtained reproduced plants were first generation transgenic plants (hereinbelow, sometimes abbreviated to "T0").

### [Example 12]

### Selection of Gene Edited Line

For confirming the presence or absence of gene recombination and edited site (site with deletion, insertion or replacement of a nucleotide) in the target gene of the first generation transgenic plants, the desired sites were amplified by PCR using the following primers: primer 7 (5'-CTCCACTTCACCACCGCAAA-3' (SEQ ID NO:32)) and primer 8 (5'-TACCGGAGATGGGTAAGCTG-3' (SEQ ID NO:33)) for the region in the RLK2 gene (Solyc03g043770).

PCR was performed using "KOD Plus Neo" manufactured and sold by TOYOBO CO., LTD., and DNA was amplified in accordance with the enclosed manual.

Next, the amplified fragments were treated with a restriction enzyme having its cleavage site in the target site of the fragments to confirm whether the amplified fragments are cleaved by the restriction enzyme or not. Specifically, *Pml*I was used for the RLK2 gene. Amplified fragments will not be cleaved by the restriction enzyme when the restriction site is changed by transgenic and editing of the gene. Occurrence of gene editing in the target gene was determined based on the non-cleavage of the amplified fragments (data not shown). Presence or absence of editing was confirmed by sequencing the nucleotide sequence of the site by the same method as described in [Example 14].

As a result, in some of the reproduced plants, editing of a sequence in the RLK2 gene was confirmed and edited lines were selected. Edited lines of the RLK2 gene were individually named "P15," "P24" and the like.

### [Example 13]

### Test for confirming TYLCV resistance of RLK2 mutated tomato

Plants (T0) of P15 line (having 141 nucleotide deletion (Mutation 1) shown in FIG. 13), a RLK2 gene-edited line selected in Example 12, were grown in an isolated green house and were self-pollinated for collecting seeds. The seeds which are transgenic progeny (T1) are sown and grown. The thus obtained T1 seedlings were inoculated with infectious clone of TYLCV-Israel strain.

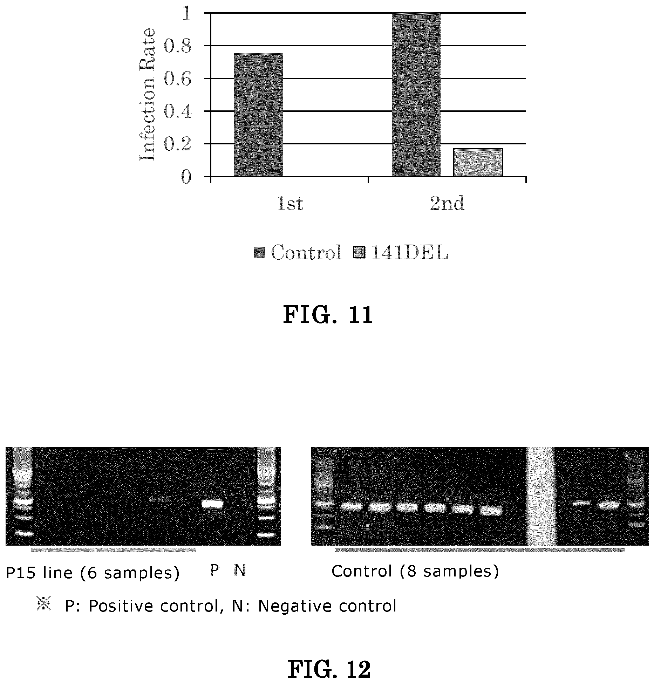

In the first inoculation test, observation of symptoms were performed on 21 days post inoculation. Regarding the P15 line, 5 plants out of 5 plants inoculated with the virus had no symptoms. On the other hand, regarding the control (wild type variety MM), 6 plants out of 8 plants inoculated with the virus showed symptoms (see FIG. 11).

Next, DNA was extracted from leaves of seedlings participating in the test and subjected to PCR analysis using TYLCV detection primer 5 (5'-CCCTCTGGAATGAAGGAACA-3') and primer 6 (5'-TTGAAAAATTGGRCTCTCAA-3'). KOD-Plus- Neo manufactured and sold by TOYOBO CO., LTD. was used as an enzyme for PCR, and PCR was performed in accordance with the enclosed manual. When the PCR products were confirmed by agarose gel electrophoresis, virus was not detected in any plants of the P15 line. On the other hand, virus was detected in 6 out of 8 control plants (see Table 3 below).

In addition, second TYLCV inoculation test was performed in the same manner as the first test using the T1 seedlings of the P15 line and control. As a result, during the observation of symptoms on 28 days post inoculation, 5 plants out of 6 plants inoculated with the virus had no symptoms. On the other hand, regarding the control (MM), all 8 plants inoculated with the virus showed symptoms. As in the first test, DNA was extracted from leaves of seedlings participating in the test and subj ected to PCR analysis using TYLCV detection primers. When the PCR products were confirmed by agarose gel electrophoresis, virus was detected in only 1 out of 6 plants of the P15 edited line, and in all of the control plants (see Table 3 and FIGS. 11 and 12).

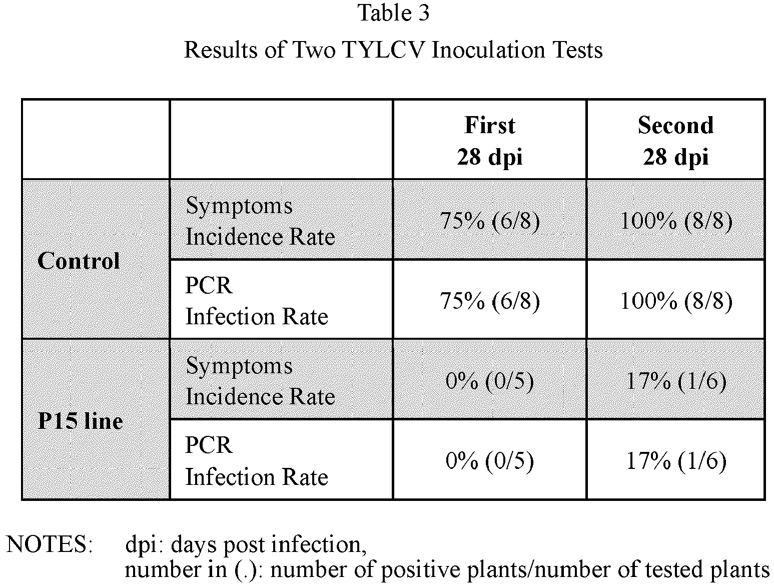

### [Example 14]

### Sequencing of TYLCV resistance RLK2 gene

A region around the RLK2 edited site (i.e., a region containing the 184th nucleotide of exon 1 (SEQ ID NO:30) of the RLK2 gene on chromosome 3 of tomato) of a TYLCV resistance T1 plant selected in Example 12 was amplified by PCR (T100 thermocycler, manufactured and sold by Bio-Rad laboratories Inc.) using the above-mentioned primer 7 and primer 8. The amplified fragments were cloned for determining the nucleotide sequence.

As a result, deletion of nucleotides in the same region was confirmed. Sequences of the mutated regions found in the RLK2 gene are shown in FIG. 13, together with the wild type nucleotide sequence of the corresponding region as set forth in SEQ ID NO:29. Specifically, the following 2 types of mutations, Mutation 1 and Mutation 2 were detected. Mutation 1 is a deletion of 141 nucleotides (SEQ ID NO:36) in exon 1 which was found in the P15 line, and the nucleotide sequence of the mutated exon 1 is shown in SEQ ID NO:34, and the nucleotide sequence of the mutated RLK2 gene is shown in SEQ ID NO:38. Mutation 2 is a deletion of 12 nucleotides (SEQ ID NO:37) in exon 1 which was found in the P24 line, and the nucleotide sequence of the mutated exon 1 is shown in SEQ ID NO:35, and the nucleotide sequence of the mutated RLK2 gene is shown in SEQ ID NO:39.

In addition, a region around the RLK2 edited site of a TYLCV resistance T1 plant selected in the same manner as in Example 12 above was amplified by PCR, and the amplified fragments were cloned for determining the nucleotide sequence. As a result, new mutation containing a deletion of 6 nucleotides was found. Sequence of the mutated regions found in the RLK2 gene is shown in FIG. 13 as Mutation 3. Mutation 3 was found in P19 line and is a deletion of 6 nucleotides (SEQ ID NO:41) in exon 1. The nucleotide sequence of the mutated exon 1 is set forth in SEQ ID NO:40 and the nucleotide sequence of the mutated RLK2 gene is set forth in SEQ ID NO:42.

### [Example 15]

### Test 1 for confirming TYLCV resistance of RLK2 mutated tomato

Plants (T0) of P24 line (having 12 nucleotide deletion (Mutation 2) shown in FIG. 13), the RLK2 gene-edited line selected in Example 12, were grown in an isolated green house and were self-pollinated for collecting seeds. The seeds which are transgenic progeny (T1) are sown and grown. The thus obtained T1 seedlings were inoculated with infectious clone of TYLCV-Israel strain. The wild type variety MM free of introduced mutation was also tested as a control.

In the inoculation test, observation of symptoms were performed on days 21 to 28 post inoculation. In the observation of symptoms, plants were defined as positive when typical symptoms of TYLCV infection, such as yellowing, curling (curving), dwarf, and/or chlorosis of the leaves, were observed. Regarding the P24 line, 7 plants out of 8 plants inoculated with the virus had no symptoms. On the other hand, regarding the control, 12 plants out of 16 plants inoculated with the virus showed symptoms (see Table 4 below).

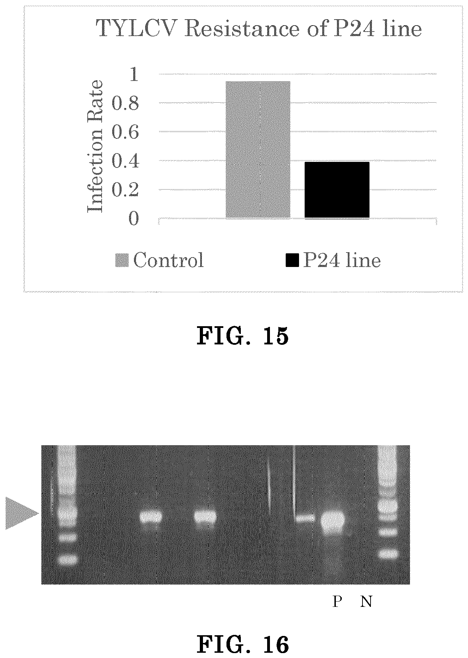

DNA was extracted from leaves of seedlings participating in the test and subjected to PCR analysis using TYLCV detection primers 5 and 6 (SEQ ID NOs: 11 and 12). When the PCR products were confirmed by agarose gel electrophoresis, virus was not detected in 3 out of 8 plants of the P24 line, but in the control, virus was detected in 15 out of 16 plants (see Table 4, and FIGS. 15 and 16).

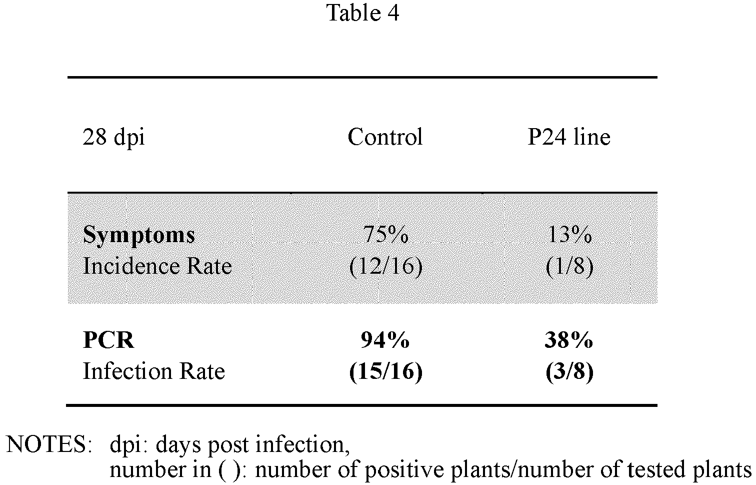

### [Example 16]

### Test 2 for confirming TYLCV resistance of RLK2 mutated tomato

The seeds which are transgenic progeny (T1) of plants (T0) of P19 line (having 6 nucleotide deletion (Mutation 3) shown in FIG. 13), the RLK2 gene-edited line selected in Example 12, were collected, sown and grown. The thus obtained T1 seedlings were inoculated with infectious clone of TYLCV-Israel strain.

In the inoculation test, observation of symptoms were performed on 21 to 28 days post inoculation. Regarding the P19 line, 4 plants out of 5 plants inoculated with the virus had no symptoms. On the other hand, regarding the control, 10 plants out of 11 plants inoculated with the virus showed symptoms (see Table 5 below).

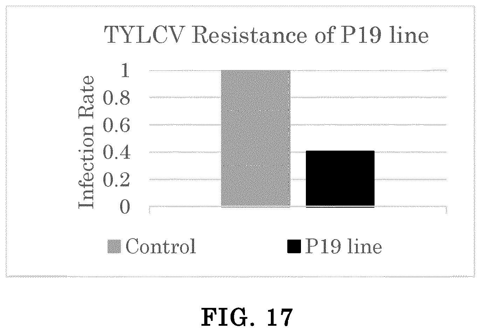

Next, DNA was extracted from leaves of seedlings subjected to the test and subjected to PCR analysis using the TYLCV detection primers 5 and 6 (SEQ ID NOs: 11 and 12). When the PCR products were confirmed by agarose gel electrophoresis, virus was detected in only 2 out of 5 plants of the P 19 line, but in the control, virus was detected in 11 out of 11 tested plants (see Table 5 and FIG. 17).

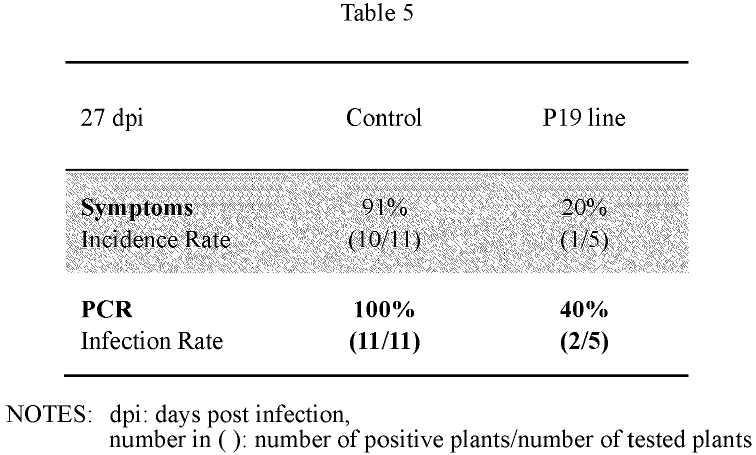

### [Example 17]

### Test 2 for confirming TYLCV resistance of DCL3 mutated tomato

DC3-2D plants (T0) (having Mutation D5 of FIG. 8 which is 2 nucleotide deletion) selected in Example 4 from plants of the "DCL3," the DCL3 edited line, was grown in an isolated green house and seeds which are transgenic progeny (T1 or T2) were sown. The thus obtained seedlings were inoculated with infectious clone of TYLCV-Israel strain by the method for the test for confirming the TYLCV resistance. Wild-type variation MM without an introduced mutation was used as a control.

In the inoculation test, observation of symptoms were performed on 21 to 28 days post inoculation. In the observation of symptoms, plants were defined as positive when typical symptoms of TYLCV infection, such as yellowing, curling (curving), dwarf, and/or chlorosis of the leaves, were observed. Regarding the DC3-2D line, 6 out of 8 inoculated plants showed no symptoms on 28 days post inoculation. On the other hand, 17 out of 18 plants inoculated with the virus showed symptoms (see Table 6 below).

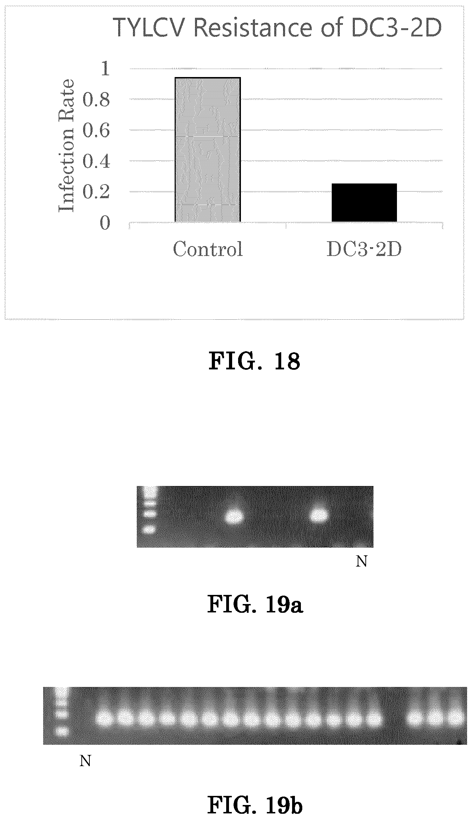

Next, DNA was extracted from leaves of seedlings participating in the test and subjected to PCR analysis using TYLCV detection primers. When the PCR products were confirmed by agarose gel electrophoresis using 1% agarose gel, virus was detected in only 2 out of 8 plants of the DC3-2D line, but in the control, virus was detected in 17 out of 18 plants (see Table 6 below and FIGS. 18, 19a and 19b).

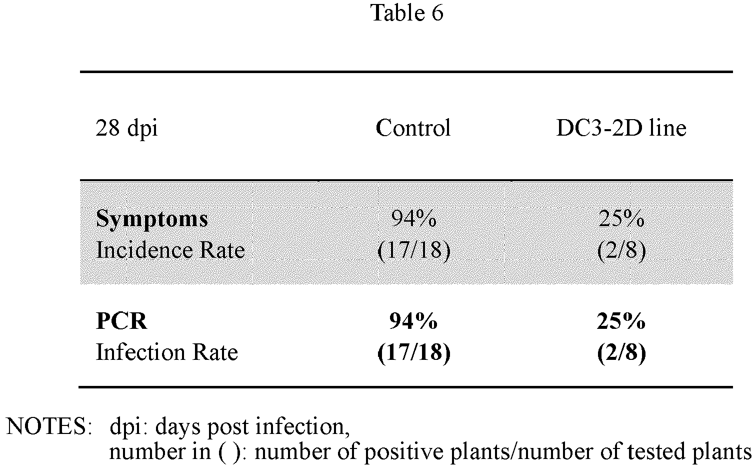

This application is an application claiming priority based on Japanese Patent Application No. 2021-034360 filed on March 4, 2021 and Japanese Patent Application No. 2021-124112 filed on July 29, 2021, and the contents described in the claims, description and drawings of the above-mentioned applications are incorporated into the present application.

### Industrial Applicability

According to the present disclosure, there is provided a virus resistant solanaceous plant, solanaceous plant cell, and method for producing the solanaceous plant, and the solanaceous plant has inhibitory properties against: infection by a virus of genus *Begomovirus* causing tomato yellow leaf curl symptoms, propagation of the infected virus, and/or expression of infection symptoms. The present disclosure is capable of solving problems mainly in agricultural fields, such as decrease in solanaceous plant yield caused by infection of the above-mentioned virus.

### Sequence Listing Free Text

SEQ ID NO: 1: cDNA sequence of DCL3 gene, 1089th to 1348th nucleotides are exon 8, and 1308th to 1327th nucleotides are target sequence
SEQ ID NO:2: Exon 8 of DCL3 gene, and 220th to 239th nucleotides are target sequence
SEQ ID NO:3: Target sequence in exon 8 of DCL3 gene
SEQ ID NO:4: cDNA sequence of 4CL06 gene, 669th to 1136th nucleotides are exon 2, and 954th to 973rd nucleotides are target sequence
SEQ ID NO:5: Exon 2 of 4CL06 gene, and 286th to 305th nucleotides are target sequence
SEQ ID NO:6: Target sequence in exon 2 of 4CL06 gene
SEQ ID NO:7: Primer 1 for detecting DCL3 gene
SEQ ID NO:8: Primer 2 for detecting DCL3 gene
SEQ ID NO:9: Primer 3 for detecting 4CL06 gene
SEQ ID NO: 10: Primer 4 for detecting 4CL06 gene
SEQ ID NO: 11: Primer 5 for detecting TYLCV
SEQ ID NO: 12: Primer 6 for detecting TYLCV
SEQ ID NO: 13 : Exon 8 of DCL3 gene with Mutation D1
SEQ ID NO: 14: Exon 8 of DCL3 gene with Mutation D2
SEQ ID NO: 15: Exon 8 of DCL3 gene with Mutation D3
SEQ ID NO: 16: Exon 8 of DCL3 gene with Mutation D4
SEQ ID NO: 17: Exon 8 of DCL3 gene with Mutation D5
SEQ ID NO: 18: Exon 8 of DCL3 gene with Mutation D6
SEQ ID NO: 19: Exon 2 of 4CL06 gene with Mutation G1
SEQ ID NO:20: Exon 2 of 4CL06 gene with Mutation G2
SEQ ID NO:21: cDNA sequence of DCL3 gene with Mutation D1 in exon 8
SEQ ID NO:22: cDNA sequence of DCL3 gene with Mutation D2 in exon 8
SEQ ID NO:23: cDNA sequence of DCL3 gene with Mutation D3 in exon 8
SEQ ID NO:24: cDNA sequence of DCL3 gene with Mutation D4 in exon 8
SEQ ID NO:25: cDNA sequence of DCL3 gene with Mutation D5 in exon 8
SEQ ID NO:26: cDNA sequence of DCL3 gene with Mutation D6 in exon 8
SEQ ID NO:27: cDNA sequence of 4CL06 gene with Mutation G1 in exon 2
SEQ ID NO:28: cDNA sequence of 4CL06 gene with Mutation G2 in exon 2
SEQ ID NO:29: cDNA sequence of RLK2 gene in which 1st to 830th nucleotides are exon 1, 202nd to 342nd nucleotides are deletion in Mutation 1, 269th to 280th nucleotides are deletion in Mutation 2, 280th to 285th nucleotides are deletion in Mutation 3, and 277th to 296th nucleotides are target sequence
SEQ ID NO:30: Exon 1 of RLK2 gene in which 202nd to 342nd nucleotides are deletion in Mutation 1, 269th to 280th nucleotides are deletion in Mutation 2, 280th to 285th nucleotides are deletion in Mutation 3, and 277th to 296th nucleotides are target sequence
SEQ ID NO:31: Target sequence in exon 1 of RLK2 gene
SEQ ID NO:32: Primer 7 for detecting RLK2 gene
SEQ ID NO:33: Primer 8 for detecting RLK2 gene
SEQ ID NO:34: Exon 1 of RLK2 gene with Mutation 1
SEQ ID NO:35: Exon 1 of RLK2 gene with Mutation 2
SEQ ID NO:36: 141 nucleotide deletion in Mutation 1
SEQ ID NO:37: 12 nucleotide deletion in Mutation 2
SEQ ID NO:38: cDNA sequence of mutated RLK2 gene of P15 line, 1st to 689th nucleotides is exon 1 with Mutation 1
SEQ ID NO:39: cDNA sequence of mutated RLK2 gene of P24 line, 1st to 818th nucleotides is exon 1 with Mutation 2
SEQ ID NO:40: Exon 1 of RLK2 gene with Mutation 3
SEQ ID NO:41: 6 nucleotide deletion in Mutation 3
SEQ ID NO:42: cDNA sequence of mutated RLK2 gene of P19 line, 1st to 824th nucleotides is exon 1 with Mutation 3

[Sequence Listing]

## Claims

1. A solanaceous plant having a mutation in at least one gene selected from a group consisting of DCL3 gene which is silencing associated dicer gene, and a gene homologous thereto; 4CL06 gene which is 4-coumarate-CoA ligase gene, and a gene homologous thereto; and RLK2 gene (Solyc03g043770) which is receptor-like kinase gene, and a gene homologous thereto exclusive of RLK1 gene (Solyc02g091840),
wherein the mutation either inhibits expression of the mutated gene or makes a protein encoded by the mutated gene to be non-functional for a virus of genus *Begomovirus* causing tomato yellow leaf curl symptoms, and
wherein the solanaceous plant has virus resistance against the virus.

2. The solanaceous plant according to claim 1, wherein the virus of genus *Begomovirus* causing tomato yellow leaf curl symptoms is Tomato yellow leaf curl virus.

3. The solanaceous plant according to claim 1 or 2, wherein the mutation is a genomic gene mutation introduced by genome editing techniques.

4. The solanaceous plant according to any one of claims 1 to 3, wherein the mutation is in the DCL3 gene which is the silencing associated dicer gene, or the gene homologous thereto.

5. The solanaceous plant according to any one of claims 1 to 3, wherein the mutation is in the 4CL06 gene which is the 4-coumarate-CoA ligase gene, or the gene homologous thereto.

6. The solanaceous plant according to claim 4 or 5, wherein the mutation is at least one type of mutation selected from (a) to (d) below:
(a) a frameshift mutation,
(b) a nonsense mutation,
(c) a loss of continuous or non-continuous 3n nucleotides (wherein n = 1 to 20), and
(d) a replacement, deletion, addition, and/or insertion of 1 or more nucleotides.

7. The solanaceous plant according to claim 4 or 6, wherein:
the DCL3 gene which is the silencing associated dicer gene has a cDNA sequence comprising a nucleotide sequence as set forth in SEQ ID NO:1, and
the gene homologous to the DCL3 gene which is the silencing associated dicer gene has a cDNA sequence comprising a nucleotide sequence which has at least 85% homology to the nucleotide sequence as set forth in SEQ ID NO: 1.

8. The solanaceous plant according to claim 7, wherein the DCL3 gene which is the silencing associated dicer gene, or the gene homologous thereto has the mutation in a region corresponding to a nucleotide sequence as set forth in SEQ ID NO:2.

9. The solanaceous plant according to claim 8, wherein the mutation is present in a region corresponding to a nucleotide sequence as set forth in SEQ ID NO:3 in the DCL3 gene, which is the silencing associated dicer gene, or the gene homologous thereto.

10. The solanaceous plant according to claim 8, wherein the region corresponding to the nucleotide sequence as set forth in SEQ ID NO:2 is mutated to a nucleotide sequence selected from those as set forth in SEQ ID NOs:21 to 26.

11. The solanaceous plant according to claim 4 or 7, wherein:
the 4CL06 gene which is the 4-coumarate-CoA ligase gene has a cDNA sequence comprising a nucleotide sequence as set forth in SEQ ID NO:4, and
the gene homologous to the 4CL06 gene which is the 4-coumarate-CoA ligase gene has a cDNA sequence comprising a nucleotide sequence which has at least 85% homology to the nucleotide sequence as set forth in SEQ ID NO:4.

12. The solanaceous plant according to claim 11, wherein the 4CL06 gene which is the 4-coumarate-CoA ligase gene, or the gene homologous thereto has the mutation in a region corresponding to a nucleotide sequence as set forth in SEQ ID NO:5.

13. The solanaceous plant according to claim 12, wherein the mutation is present in a region corresponding to a nucleotide sequence as set forth in SEQ ID NO:6 in the 4CL06 gene which is 4-coumarate-CoA ligase gene, or the gene homologous thereto.

14. The solanaceous plant according to claim 12, wherein the region corresponding to the nucleotide sequence as set forth in SEQ ID NO:5 is mutated to a nucleotide sequence selected from those as set forth in SEQ ID NOs:27 and 28.

15. The solanaceous plant according to any one of claims 1 to 3, wherein the mutation is in the RLK2 gene (Solyc03g043770) which is the receptor-like kinase gene, or the gene homologous thereto.

16. The solanaceous plant according to claim 15, wherein at least one gene selected from a group consisting of the RLK2 gene (Solyc03g043770) which is receptor-like kinase gene, and the gene homologous thereto has a mutation, and the mutation is at least one type of mutation selected from (c) and (d) below:
(c) a loss of continuous or non-continuous 3n nucleotides (wherein n = 1 to 99), and
(d) a replacement, deletion, addition, and/or insertion of 1 or more nucleotides.

17. The solanaceous plant according to claim 16, wherein the mutation is at least one type of mutation selected from (c) and (d') below:
(c) a loss of continuous or non-continuous 3n nucleotides (wherein n = 1 to 99), and
(d') an insertion of continuous or non-continuous 3n nucleotides (wherein n = 1 to 2).

18. The solanaceous plant according to any one of claims 15 to 17, wherein:
the RLK2 gene which is receptor-like kinase gene has a cDNA sequence comprising a nucleotide sequence as set forth in SEQ ID NO:29, and
the gene homologous to the RLK2 gene which is the receptor-like kinase gene has a cDNA sequence comprising a nucleotide sequence which has at least 85% homology to the nucleotide sequence as set forth in SEQ ID NO:29.

19. The solanaceous plant according to claim 18, wherein the RLK2 gene which is the receptor-like kinase gene or the gene homologous thereto has the mutation in a region corresponding to a nucleotide sequence as set forth in SEQ ID NO:30.

20. The solanaceous plant according to claim 19, wherein the mutation is present in a region corresponding to a nucleotide sequence as set forth in SEQ ID NO:36.

21. The solanaceous plant according to claim 19 or 20, wherein the region corresponding to the nucleotide sequence as set forth in SEQ ID NO:30 is mutated to a nucleotide sequence selected from those as set forth in SEQ ID NOs:34, 35 and 40.

22. The solanaceous plant according to any one of claims 1 to 21 which is a tomato.

23. Apart of the solanaceous plant according to any one of claims 1 to 22.

24. The part of the solanaceous plant according to claim 23 which is a fruit.

25. The part of the solanaceous plant according to claim 23 which is a seed.

26. A processed material of the solanaceous plant or the part thereof according to any one of claims 1 to 22.

27. The processed material according to claim 26 which is edible.

28. A solanaceous plant cell having a mutation in at least one gene selected from a group consisting of DCL3 gene which is silencing associated dicer gene, and a gene homologous thereto; 4CL06 gene which is 4-coumarate-CoA ligase gene, and a gene homologous thereto; and RLK2 gene (Solyc03g043770) which is receptor-like kinase gene, and a gene homologous thereto exclusive of RLK1 gene (Solyc02g091840),
wherein the mutation either inhibits expression of the mutated gene or makes a protein encoded by the mutated gene to be non-functional for a virus of genus *Begomovirus* causing tomato yellow leaf curl symptoms, and
wherein the solanaceous plant cell has virus resistance against the virus.

29. The solanaceous plant cell according to claim 28, wherein the virus of genus *Begomovirus* causing tomato yellow leaf curl symptoms is tomato yellow leaf curl virus.

30. The solanaceous plant cell according to claim 28 or 29, wherein the solanaceous plant is a tomato.

31. A solanaceous plant and a part thereof comprising the solanaceous plant cell according to any one of claims 28 to 30, and having virus resistance against a virus of genus *Begomovirus* causing tomato yellow leaf curl symptoms.

32. A method for producing a virus resistant solanaceous plant which is resistant to a virus of genus *Begomovirus* causing tomato yellow leaf curl symptoms, the method comprising:
selecting at least one gene from a group consisting of DCL3 gene which is silencing associated dicer gene, and a gene homologous thereto; 4CL06 gene which is 4-coumarate-CoA ligase gene, and a gene homologous thereto; and RLK2 gene (Solyc03g043770) which is receptor-like kinase gene, and a gene homologous thereto exclusive of RLK1 gene (Solyc02g091840),
introducing a mutation into the selected gene in a genome, wherein the introduced mutation is either a mutation inhibiting an expression of the mutated gene or a mutation making a protein encoded by the mutated gene to be non-functional for a virus of genus *Begomovirus* causing tomato yellow leaf curl symptoms; and
selecting a solanaceous plant having resistance to the virus.

33. The method for producing a virus resistant solanaceous plant according to claim 32, wherein the virus of genus *Begomovirus* causing tomato yellow leaf curl symptoms is Tomato yellow leaf curl virus.

34. The method for producing a virus resistant solanaceous plant according to claim 32 or 33, wherein the mutation is introduced into the gene in the genome by genome editing techniques.

35. The method for producing a virus resistant solanaceous plant according to any one of claims 32 to 34, wherein the mutation is introduced into the DCL3 gene which is the silencing associated dicer gene, or the gene homologous thereto.

36. The method for producing a virus resistant solanaceous plant according to any one of claims 32 to 34, wherein the mutation is introduced into the 4CL06 gene which is the 4-coumarate-CoA ligase gene, or the gene homologous thereto.

37. The method for producing a virus resistant solanaceous plant according to claim 35 or 36, wherein the mutation is at least one type of mutation selected from (a) to (d) below:
(a) a frameshift mutation,
(b) a nonsense mutation,
(c) a loss of continuous or non-continuous 3n nucleotides(wherein n = 1 to 20), and
(d) a replacement, deletion, addition, and/or insertion of 1 or more nucleotides.

38. The method for producing a virus resistant solanaceous plant according to claim 35 or 37, wherein:
the DCL3 gene which is the silencing associated dicer gene has a cDNA sequence comprising a nucleotide sequence as set forth in SEQ ID NO:1, and
the gene homologous to the DCL3 gene which is the silencing associated dicer gene has a cDNA sequence comprising a nucleotide sequence which has at least 85% homology to the nucleotide sequence as set forth in SEQ ID NO: 1.

39. The method for producing a virus resistant solanaceous plant according to claim 38, wherein the mutation is introduced into the DCL3 gene which is the silencing associated dicer gene, or the gene homologous thereto at a region corresponding to a nucleotide sequence as set forth in SEQ ID NO:2.

40. The method for producing a virus resistant solanaceous plant according to claim 39, wherein the mutation is introduced into a region corresponding to a nucleotide sequence as set forth in SEQ ID NO:3 in the DCL3 gene which is the silencing associated dicer gene, or the gene homologous thereto.

41. The method for producing a virus resistant solanaceous plant according to claim 39, wherein the mutation is introduced so that the region corresponding to the nucleotide sequence as set forth in SEQ ID NO:2 is changed to a nucleotide sequence selected from those as set forth in SEQ ID NOs:21 to 26.

42. The method for producing a virus resistant solanaceous plant according to claim 36 or 37, wherein the 4CL06 gene which is the 4-coumarate-CoA ligase gene has a cDNA sequence comprising a nucleotide sequence as set forth in SEQ ID NO:4, and
the gene homologous to the 4CL06 gene which is the 4-coumarate-CoA ligase gene has a cDNA sequence comprising a nucleotide sequence which has at least 85% homology to the nucleotide sequence as set forth in SEQ ID NO:4.

43. The method for producing a virus resistant solanaceous plant according to claim 42, wherein the mutation is introduced into a region corresponding to a nucleotide sequence as set forth in SEQ ID NO:5 in the 4CL06 gene which is the 4-coumarate-CoA ligase gene, or the gene homologous thereto.

44. The method for producing a virus resistant solanaceous plant according to claim 43, wherein the mutation is introduced into a region corresponding to a nucleotide sequence as set forth in SEQ ID NO:6 in the 4CL06 gene which is the 4-coumarate-CoA ligase gene, or the gene homologous thereto.

45. The method for producing a virus resistant solanaceous plant according to claim 43, wherein the mutation is introduced so that the region corresponding to the nucleotide sequence as set forth in SEQ ID NO:5 is changed to a nucleotide sequence set forth in SEQ ID NO:27 or 28.

46. The method for producing a virus resistant solanaceous plant according to any one of claims 32 to 34, wherein the mutation is introduced into the RLK2 gene (Solyc03g043770) which is receptor-like kinase gene, and a gene homologous thereto.

47. The method for producing a virus resistant solanaceous plant according to claim 46, wherein the mutation is at least one type of mutation selected from (c) and (d) below:
(c) a loss of continuous or non-continuous 3n nucleotides (wherein n = 1 to 99), and
(d) a replacement, deletion, addition, and/or insertion of 1 or more nucleotides.

48. The method for producing a virus resistant solanaceous plant according to claim 47, wherein the mutation is at least one type of mutation selected from (c) and (d') below:
(c) a loss of continuous or non-continuous 3n nucleotides (wherein n = 1 to 99), and
(d') an insertion of continuous or non-continuous 3n nucleotides (wherein n = 1 to 2).

49. The method for producing a virus resistant solanaceous plant according to any one of claims 46 to 48, wherein:
the RLK2 gene which is receptor-like kinase gene has a cDNA sequence comprising a nucleotide sequence as set forth in SEQ ID NO:29, and
the gene homologous to the RLK2 gene which is the receptor-like kinase gene has a cDNA sequence comprising a nucleotide sequence which has at least 85% homology to the nucleotide sequence as set forth in SEQ ID NO:29.

50. The method for producing a virus resistant solanaceous plant according to claim 49, wherein the mutation is introduced into the RLK2 gene which is receptor-like kinase gene, and a gene homologous thereto at a region corresponding to a nucleotide sequence as set forth in SEQ ID NO:30.

51. The method for producing a virus resistant solanaceous plant according to claim 50, wherein the mutation is introduced into a region corresponding to a nucleotide sequence as set forth in SEQ ID NO:36.

52. The method for producing a virus resistant solanaceous plant according to claim 50 or 51, wherein the mutation is introduced so that the region corresponding to the nucleotide sequence as set forth in SEQ ID NO:30 is changed to a nucleotide sequence as set forth in SEQ ID NOs:34, 35 or 40.

53. The method for producing a virus resistant solanaceous plant according to any one of claims 32 to 52, wherein the solanaceous plant is a tomato.

54. A virus resistant solanaceous plant obtained by the production method according to any one of claims 32 to 53.

55. A method for producing a bred progeny of a virus resistant solanaceous plant which is resistant to a virus of genus *Begomovirus* which causes tomato yellow leaf curl symptoms, the method comprising: self-pollination or cross-pollination of either a virus resistant solanaceous plant obtained by the production method according to any one of claims 32 to 53 or a progeny of the virus resistant solanaceous plant.

56. A virus resistant solanaceous plant resistant to a virus of genus *Begomovirus* which causes tomato yellow leaf curl symptoms, the solanaceous plant being obtained by the production method of claim 55.
